# EUROPEAN PATENT SPECIFICATION

(11) **EP 2 809 341 B1**
(45) Date of publication and mention of the grant of the patent: **30.12.2020**
(21) Application number: 13743239.9
(22) Date of filing: 01.02.2013
(51) Int. Cl.: A61K 38/43, A61K 38/54, A61P 17/02

(54) **ENZYME COMPOSITIONS AND USE THEREOF FOR WOUND HEALING**
ENZYMZUSAMMENSETZUNGEN UND ANWENDUNG DAVON ZUR WUNDHEILUNG
COMPOSITIONS ENZYMATIQUES ET LEUR UTILISATION POUR LA CICATRISATION

(30) Priority: 02.02.2012 US 201261594015 P
(43) Date of publication of application: 10.12.2014
(73) Proprietor: Galenagen, LLC, Rye Brook, NY 10573 (US)
(72) Inventor: FALLON, Joan, M., Rye Brook NY 10573 (US); HEIL, Matthew, F., Rye Brook NY 10573 (US); SZIGETHY, James, Rye Brook NY 10573 (US); FALLON, James, Rye Brook NY 10573 (US)
(74) Representative: HGF
(86) International application number: PCT/US2013/024453
(87) International publication number: WO 2013/116732

(56) References cited:
- WO-A1-84/02846
- WO-A1-2009/155689
- WO-A1-2010/080835
- WO-A2-02/051352
- WO-A2-02/051436
- WO-A2-2007/074454
- US-A1- 2005 036 950
- SHELBY R W ET AL: "Enzymatic debridement with activated whole pancreas", AMERICAN JOURNAL OF SURGERY, PAUL HOEBER, NEW YORK, NY, US, vol. 96, no. 4, 1 October 1958 (1958-10-01), pages 545-549, XP026485068, ISSN: 0002-9610, DOI: 10.1016/0002-9610(58)90972-3 [retrieved on 1958-10-01]
- MARCUS FERRONE ET AL: "Pancreatic Enzyme Pharmacotherapy", PHARMACOTHERAPY, vol. 27, no. 6, 1 January 2007 (2007-01-01), pages 910-920, XP055090867,

## Description

### BACKGROUND OF THE INVENTION

Wound healing in tissues is a complex reparative process. If a wound does not heal in an orderly or timely sequence, or if the healing process does not result in structural integrity, then the wound is considered chronic. In spite of advances in recombinant growth factors and bioengineered skin, up to 50% of chronic wounds that have been present for more than a year remain resistant to treatment.

Skin ulcers are probably the most common types of chronic wounds. These wounds can be created or perpetuated by many factors, including vascular insufficiency, either venous or arterial, prolonged inflammation, pressure necrosis, physical agents, infection, and cancer. Seventy percent of skin wounds, however, are due to pressure ulcers, diabetic foot ulcers, and venous ulcers. Normally, antibiotics like mupirocin, metronidazole, polymyxin B, Neosporin, or bacitracin are applied to the wounded area to avoid bacterial infestation that may further deteriorate the condition if it occurs. However, such practice may be able to clear bacterial infestation but not necessarily lead to healing of the wound. Moreover, these chemically synthesized drugs tend to cause tolerance or side effect onto the users. Chronic wounds and their treatment are a huge burden on the healthcare system, in terms of cost, time and attention of care required. The loss in productivity and decreased quality of life is immeasurable.

Under normal circumstances, the process of acute wound healing can be broken down into three phases. An initial inflammatory phase, which is followed by robust tissue remodeling and proliferation (the proliferative phase), and is succeeded by a maturational phase wherein re-epithelialization, dermal angiogenesis and wound closure ensues. Re-epithelialization involves the migration and proliferation of epithelial tissue, primarily keratinocytes. Angiogenesis is the growth of new blood vessels from pre-existing conduits, and is regulated by a panoply of soluble cytokines including growth factor polypeptides, as well as cell-cell and cell-matrix interactions. Chronic wounds exhibit a different healing profile from normal acute wounds in that they generally remain in an inflamed state for protracted periods of time. Non-healing wounds can most commonly be observed amongst people with diabetes, venous stasis disease, and in those patients who are immobilized.

Nothing in the Background of the Invention should be construed as an admission of prior art.

### SUMMARY OF THE INVENTION

This disclosure relates to the treatment of wounds, with the use of a pharmaceutical composition comprising one or more digestive enzymes, such as pancreatic or other digestive-tract enzymes (*e.g*., porcine pancreatic enzymes) or plant-, fungal-, or microorganism-derived enzymes, that break down components of food. As used herein, a pharmaceutical composition can be used for human or veterinary indications. Accordingly, the pharmaceutical compositions may be useful for therapeutic treatment of human or other mammalian populations (*e.g*., pig, horse, cow, sheep, goat, monkey, rat, mouse, cat, dog, llama, panda, lion, tiger, hippopotamus, rhinoceros, giraffe, hamster, gerbil, *etc.*) or of bird populations (*e.g.,* duck, goose, chicken, turkey, ostrich, *etc.*)*.* Mammals to be treated may also include all Therians (mammals which give live birth) and Monotremes (egg laying mammals). In addition the present methods can be used for all other forms of vertebrates and invertebrates including, but not limited to Fish, Reptiles, and Amphibians.

The invention provides a topical pharmaceutical compositon for use in wound healing according to claim 1.

The pharmaceutical compositons can be used on their own, and/or in combination with other wound healing agents. Accordingly, it is an object of the present disclosure to provide a method for treating wounds in a bird or a mammal, comprising administering to the bird or mammal a therapeutically effective amount of a pharmaceutical composition comprising one or more digestive enzymes and one or more pharmaceutically acceptable excipients. In some embodiments, the digestive enzymes comprise a combination of enzymes such as, for example, proteases, amylases cellulases, sucrases, maltases, papain, lipases. In some embodiments, the digestive enzymes comprise one or more pancreatic enzymes. The digestive enzymes may be derived from an animal source, a microbial source, a plant source, a fungal source, or are synthetically prepared. In certain embodiments, the enzymes are porcine-derived. In some embodiments, the animal source is a pig pancreas.

In another embodiment, the therapeutic composition may be pancreatin.

In another embodiment, the therapeutic composition may be a solid form of pancreatin.

In another embodiment, the therapeutic composition may be a crystalline form of pancreatin.

In one non-limiting example, the composition comprises proteases, lipases and amylases in a base of white petrolatum. In some embodiments, a pharmaceutical composition comprises at least one amylase, a mixture of proteases comprising chymotrypsin and trypsin, and at least one lipase.

In embodiments, a pharmaceutical preparation comprises protease, lipase and amylase.

In some embodiments, the compositions may comprise one or more additional wound healing agents. Alternatively, in other embodiments, the compositions may be administered with one or more additional wound healing agents. In some embodiments, the pharmaceutical composition is a dosage formulation for topical administration where the composition is an aqueous solution, emulsion, cream, ointment, suspension, gel, lotion, liposomal suspension, or a combination of any thereof.

Further disclosed herein is a method for promoting wound healing and/or reducing scarring in an individual with a wound, comprising administering a pharmaceutical composition comprising one or more digestive enzymes to the individual. The wound can be an acute wound or a chronic wound (e.g., a surgical wound or a traumatic wound).

Scarring may be reduced by at least about 2-fold, about 3-fold,
about 4-fold, about 5-fold, about 7.5-fold, about 10-fold, about 15-fold, about 20-fold, about 25-fold or more compared a subject treated with a placebo.
Scarring may be reduced by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

Scarring may be reduced by at least 2-fold, about 3-fold,
about 4-fold, about 5-fold, about 7.5-fold, about 10-fold, about 15-fold, about 20-fold, about 25-fold or more compared to a subject not receiving treatment with a composition described herein. Scarring may be reduced by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

Further disclosed are methods of applying the composition above to a wound
where the composition is useful for stimulating epidermal cells, causing short term fibrosis deposits, preventing re-opening of wounds, recruiting white blood cells to help growth factor and immune system activation (enzyme antibiotic effect), inducing greater re-growth of hair, reducing alopecia, enhances epidermal restoration and integrity beyond that of the normal restorative process, or a combination thereof.

Disclosed herein is topical wound-healing pharmaceutical composition, comprising a therapeutically effective amount of one or more digestive enzymes and one or more excipients, wherein said digestive enzymes comprise from about 25 to about 700,000 U.S.P units protease, about 2 to about 100,000 U.S.P units lipase and about 25 to about 400,000 U.S.P units of amylase, wherein said therapeutically effective amount of said one or more digestive enzymes is sufficient to induce a favorable epidermal physiological response.

The epidermal physiological response may comprise epidermal
hyperplasia, short term fibrosis deposits, recruitment of white blood cells and/or immune system activation.

A therapeutically effective amount of one or more digestive
enzymes may consist essentially of protease, lipase and amylase.

In one example, the composition is not used for treating a *S. aureus* or *E. coli* infection.

In one embodiment, the composition is pancreatin. In one embodiment, the one or more digestive enzymes further comprise one or more enzymes selected from the group consisting of cellulases, sucrases, maltases, and papain. In one embodiment, the one or more digestive enzymes comprise one or more pancreatic enzymes. In one embodiment, the one or more of the digestive enzymes comprise porcine-derived enzymes. In one embodiment, the protease comprises chymotrypsin and trypsin. In one embodiment, the one or more digestive enzymes are, independently, derived from an animal source, a microbial source, a plant source, a fungal source, or are synthetically prepared. In one embodiment, the composition comprises at least one amylase, a mixture of proteases comprising chymotrypsin and trypsin, and at least one lipase.
The ratio (in U.S.P. units) of proteases to lipase to amylase is 7:1: 4.

In one embodiment, the composition comprises about 122,130 U.S.P units protease about 17,110 U.S.P. units lipase and about 73,750 U.S.P. units amylase in a base of about 30 grams of white petrolatum.

In one embodiment, the composition comprises about 238,050 U.S.P units protease about 33,350 U.S.P. units lipase and about 143,750 U.S.P. units amylase in a base of about 30 grams of white petrolatum.

In one embodiment, the composition comprises about 459,540 U.S.P. units protease, about 64,380 U.S.P. units lipase and about 277,500 U.S.P. units amylase in a base of about 30 grams of white petrolatum.

In one embodiment, the composition stimulates epidermal cells, causes short term fibrosis deposits, prevents re-opening of wounds, recruits white blood cells to help growth factor and immune system activation (enzyme antibiotic effect), induces greater re-growth of hair, reduces alopecia, enhances epidermal restoration and integrity beyond that of the normal restorative process, or a combination thereof.

In another embodiment, the composition does not cause an allergic reaction, scarring, biological damage, burns, or a combination thereof.

The composition may be a dosage formulation selected from the group consisting of: creams, lotions, emulsions, powders, liquids, gels, and a combination of any thereof.

The one or more excipients may be water, saline, Ringer's solution, dextrose solution, and solutions of ethanol, glucose, sucrose, dextran, mannose, mannitol, sorbitol, polyethylene glycol (PEG), phosphate, acetate, gelatin, collagen, CARBOPOL®, vegetable oils, white petrolatum or a combination thereof.

A composition may further comprise one or more suitable preservatives, stabilizers, antioxidants, antimicrobials, buffering agents, or a combination thereof.

Disclosed herein is a method of healing a wound in a subject comprising applying a topical pharmaceutical composition for wound healing, comprising a therapeutically effective amount of one or more digestive enzymes and one or more excipients to the wound, wherein said digestive enzymes comprise from about 25 to about 700,000 U.S.P. units protease, about 2 to about 100,000 U.S.P. lipase and about 25 to about 400,000 U.S.P. units of amylase, wherein said therapeutically effective amount of said one or more digestive enzymes is sufficient to induce a favorable epidermal physiological response.

A method of healing a wound in a subject comprising applying a topical pharmaceutical composition for wound healing comprising a therapeutically effective amount of one or more digestive enzymes and optionally one or more excipients, wherein said digestive enzymes comprise at least about 100,000 U.S.P. units protease at least about 15,000 U.S.P. units lipase and at least about 70,000 U.S.P. units of amylase.

The digestive enzymes may comprise at least about 200,000 U.S.P.
units protease at least about 30,000 U.S.P. units lipase and at least about 140,000 U.S.P. units of amylase.

The digestive enzymes may comprise at least about 450,000 U.S.P.
units protease at least about 60,000 U.S.P. units lipase and at least about 270,000 U.S.P. units of amylase.

The digestive enzymes may comprise at least about 122,000 U.S.P.
units protease at least about 17,000 U.S.P. units lipase and at least about 73,000 U.S.P. units of amylase.

The digestive enzymes may comprise at least about 238,000 U.S.P.
units protease at least about 33,000 U.S.P. units lipase and at least about 143,000 U.S.P. units of amylase.

The digestive enzymes may comprise at least about 459,000 U.S.P.
units protease at least about 64,000 U.S.P. units lipase and at least about 277,000 U.S.P. units of amylase.

The therapeutically effective amount of said one or more
digestive enzymes may be sufficient to induce a favourable epidermal physiological response.

The ratio (in U.S.P. units) of proteases to lipase to amylase in the
composition may be 7: 1: 4.

The one or more excipient may comprise white petrolatum.

The compositon may consist essentially of protease, lipase and
amylase. The composition may comprise pancreatin. The digestive enzymes in the composition may consist essentially of protease, amylase and lipase.

The subject may exhibit at least about 2X faster improvement in
wound healing following administration of said composition comprising digestive enzymes compared to a subject treated with a placebo.

The subject may exhibit at least about a 2X faster improvement in wound healing following administration of said composition compared to a subject not treated with said composition.

An epidermal physiological response produced by administration of such compositions may comprise epidermal hyperplasia, short time fibrosis deposits, recruitment of white blood cells and/or immune system activation.

Disclosed herein is a method for stimulating epidermal cells, causing short term fibrosis deposits, preventing re-opening of wounds, recruiting white blood cells to help growth factor and immune system activation (enzyme antibiotic effect), inducing re-growth of hair, reducing alopecia, enhancing epidermal restoration and integrity beyond that of the normal restorative process, or a combination thereof in a subject comprising contacting a wound with a therapeutically effective amount of a composition comprising one or more digestive enzymes and one or more excipients, wherein said digestive enzymes comprise from about 25 to about 700,000 U.S.P. units protease, and about 2 to about 100,000 U.S.P. units lipase, and about 25 to about 400,000 U.S.P. units of amylase

Disclosed herein is a method of healing a wound in a subject comprising applying a topical pharmaceutical composition for wound healing comprising a therapeutically effective amount of one or more digestive enzymes and optionally one or more excipients, wherein said digestive enzymes comprise at least about 100,000 U.S.P. units protease, at least about 15,000 U.S.P. units lipase, and at least about 70,000 U.S.P. units of amylase.

The digestive enzymes may comprise at least about 200,000 U.S.P.
units protease, at least 30,000 U.S.P. units lipase, and at least about 140,000 U.S.P. units of amylase.

The digestive enzymes may comprise at least about 450,000
U.S.P. units protease, at least about 60,000 U.S.P. units lipase, and at least about 270,000 U.S.P. units of amylase.

The digestive enzymes may comprise at least about 122,000
U.S.P. units protease, at least about 17,000 U.S.P. units lipase, and at least about 73,000 U.S.P. units of amylase.

The digestive enzymes may comprise at least about 238,000
U.S.P. units protease, at least about 33,000 U.S.P. units lipase, at at least about 143,000 U.S.P. units of amylase.

The digestive enzymes may comprise at least about 459,000
U.S.P. units protease, at least about 64,000 U.S.P. units lipase, and at least about 277,000 U.S.P. units of amylase.

The said therapeutically effective amount of said one or
more digestive enzymes may be sufficient to induce a favourable epidermal physiological response.

Disclosed herein is a method of promoting wound healing by administering to a subject a composition consisting essentially of one or more digestive enzymes and one or more excipients, wherein said digestive enzymes comprise from about 25 to about 700,000 U.S.P. units protease, and about 2 to about 100,000 U.S.P. units lipase, and about 25 to about 400,000 U.S.P. units of amylase in a base of white petrolatum, wherein the scarring is reduced by at least about 2-fold compared to administering a placebo.

In one aspect of any of the compositions and methods disclosed herein, the ratio (in U.S.P. units) of proteases to lipase to amylase in the composition may be 7: 1:4.

The digestive enzymes may comprise at least about 105,000 U.S.P.
units protease, at least about 15,000 U.S.P. units lipase, and at least about 60,000 U.S.P. units of amylase.

The digestive enzymes may comprise at least about 210,000
U.S.P. units protease, at least about 30,000 U.S.P. units lipase, and at least about 120,000 U.S.P. units of amylase.

The digestive enzyme may comprise at least about 119,000
U.S.P. units protease, at least about 17,000 U.S.P. units lipase, and at least about 68,000 U.S.P. units of amylase.

The digestive enzymes may comprise at least about 224,000
U.S.P. units protease, at least about 33,000 U.S.P. units lipase, and at least about 132,000 U.S.P. units of amylase.

### BRIEF DESCRIPTION OF THE DRAWINGS

The novel features of the compositions for use are set forth with particularity in the appended claims. A better understanding of the features and advantages of the present embodiments will be obtained by reference to the following detailed description that sets forth illustrative examples, in which the principles of the compositions and methods disclosed herein are utilized, and the accompanying drawings of which:

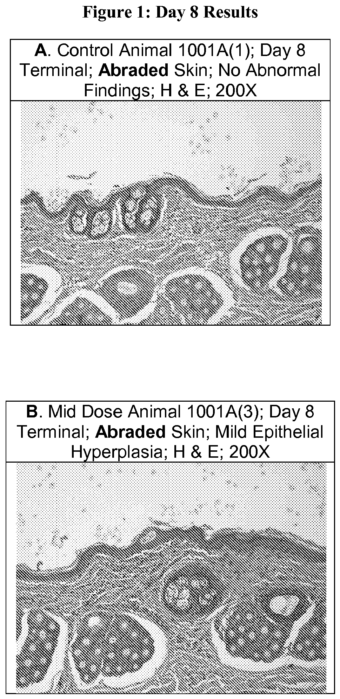

**Figures 1A-B** illustrate representative results of treatment of wounds at day 8 of the study in animal 1001A. **Figure 1A** provides an H&E stain of control animal 1001A(1) on day 8; abraded skin was observed with no abnormal finding. **Figure 1B** provides an H&E stainof mid dose animal 1001A(3) on day 8; abraded skin was observed with mild epithelial hyperplasia.

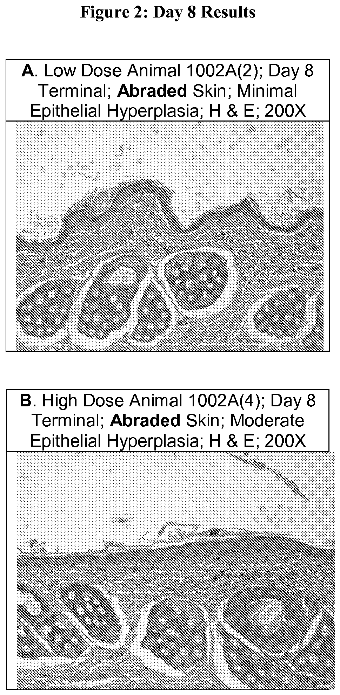

**Figures 2A-B** illustrate representative results of treatment of wounds at day 8 of the study in animal 1002A. **Figure 2A** provides an H&E stain of low dose animal 1002A(2) on day 8; abraded skin was observed with minimal epithelia hyperplasia. **Figure 2B** provides an H&E stain of high dose animal 1002A(4) on day 8; abraded skin was observed with mild epithelial hyperplasia.

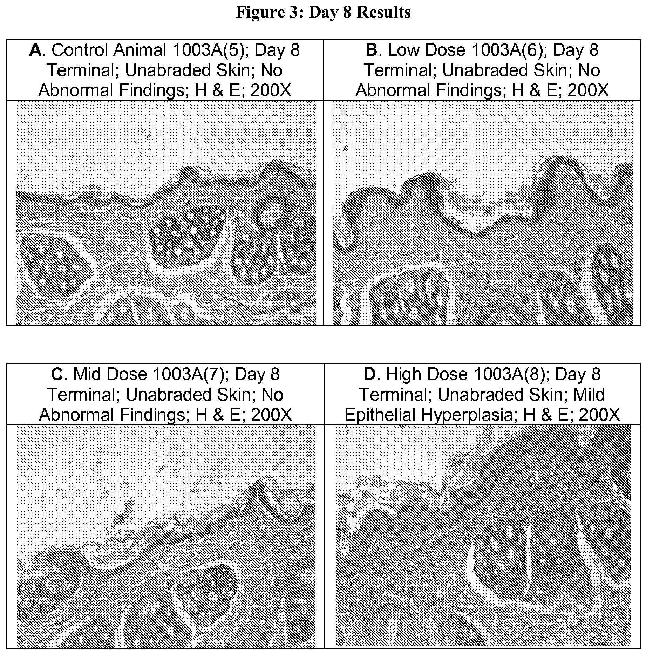

**Figures 3A-D** illustrate representative results of treatment of wounds at day 8 of the study in animal 1003A. **Figure 3A** provides an H&E stain of control animal 1003A(5) on day 8; unabraded skin was observed with no abnormal findings. **Figure 3B** provides an H&E stain of low dose animal 1003A(6) on day 8; unabraded skin was observed with no abnormal findings. **Figure 3C** provides an H&E stain of mid dose animal 1003A(7) on day 8; unabraded skin was observed with no abnormal findings. **Figure 3D** provides an H&E stain of high dose animal 1003A(8) on day 8; unabraded skin was observed with mild epithelial hyperplasia.

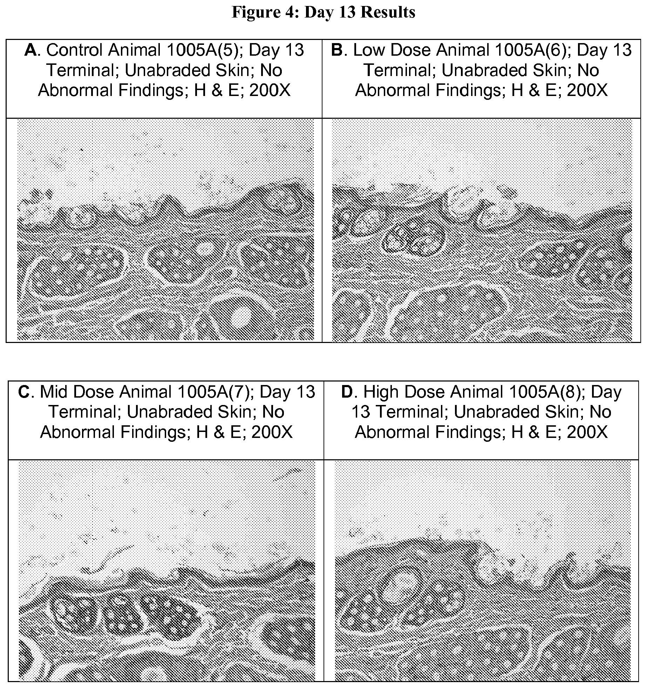

**Figures 4A-D** illustrate representative results of treatment of wounds at day 13 of the study in animal 1005A. **Figure 4A** provides an H&E stain of control animal 1005A(5) on day 13; unabraded skin was observed with no abnormal findings. **Figure 4B** provides an H&E stain of low dose animal 1005A(6) on day 13; unabraded skin was observed with no abnormal findings. **Figure 4C** provides an H&E stain of mid dose animal 1005A(7) on day 13; unabraded skin was observed with no abnormal findings. **Figure 4D** provides an H&E stain of high dose animal 1005A(8) on day 13; unabraded skin was observed with no abnormal findings; 200X resolution.

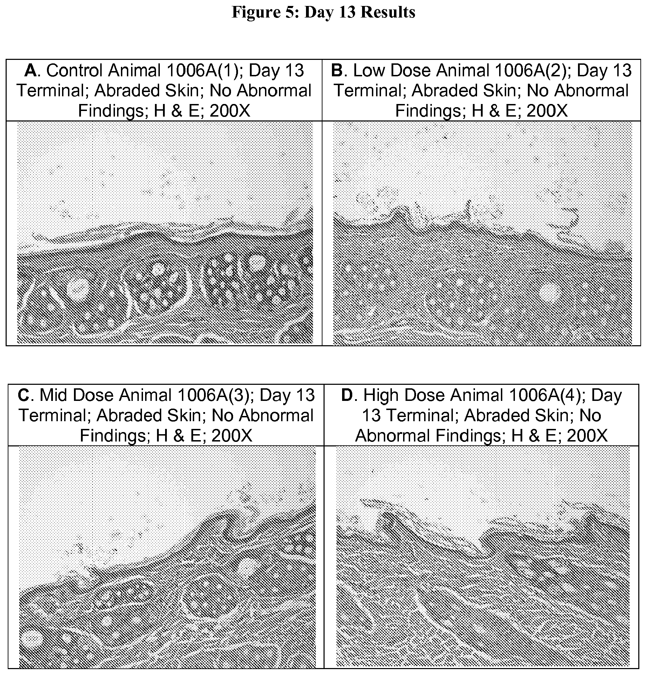

**Figures 5A-D** illustrate representative results of treatment of wounds at day 13 of the study in animal 1006A. **Figure 5A** provides an H&E stain of control animal 1006A(1) on day 13; abraded skin was observed with no abnormal findings. **Figure 5B** provides an H&E stain of low dose animal 1006A(2) on day 13; abraded skin was observed with no abnormal findings;. **Figure 5C** provides an H&E stain of mid dose animal 1006A(1) on day 13; abraded skin was observed with no abnormal findings. **Figure 5D** provides an H&E stain of high dose animal 1006A(4) on day 13; unabraded skin was observed with no abnormal findings.

### DETAILED DESCRIPTION OF THE INVENTION

The present inventors found for the first time that the enzyme compositions described herein were effective in promoting healing of wounds. Furthermore, the enzyme compositions may stimulate epidermal cells, causing short term fibrosis deposits, preventing re-opening of wounds, recruiting white blood cells to help growth factor and immune system activation (enzyme antibiotic effect), inducing greater re-growth of hair, reducing alopecia, enhancing epidermal restoration and integrity beyond that of the normal restorative process, or a combination thereof.

Described herein is a pharmaceutical composition, comprising porcine-derived proteases, lipases, and amylases, and with one or more pharmaceutically acceptable excipients or carriers.

Described herein is a method for wound healing, comprising the administration to a subject in need thereof of a therapeutically effective amount of composition described herein.

The term "administration" or "administering" refers to a method of giving a dosage of a composition or pharmaceutical composition to a subject or patient.

As used herein, a "subject" or "patient" or "individual" means a human or a non-human mammal, *e.g*., a dog, a cat, a mouse, a rat, a cow, a sheep, a pig, a goat, a non-human primate or a bird, (*e.g.,* a chicken, a turkey, an ostrich, *etc.*) as well as any other vertebrate or invertebrate. The term "mammal" is used in its usual biological sense. Thus, it specifically includes humans, cattle, horses, dogs, and cats, but also includes many other species including, but not limited to, a llama, panda, lion, tiger, hippopotamus, rhinoceros, giraffe, rodent (*e.g.,* mice, rats, rabbits, *etc.*)*,* or a primate (*e.g.,* monkeys, gorillas, chimpanzees, *etc.*) and all other forms including all Therians and Monotremes. In one embodiment, a mammal to be treated is a human.

"Treat," "treatment," or "treating," as used herein refers to administering a pharmaceutical composition for therapeutic purposes. The term "therapeutic treatment" refers to administering treatment to a patient thus causing a therapeutically beneficial effect.

By "therapeutically effective amount" or "pharmaceutically effective amount" is typically one which is sufficient to achieve the desired effect and may vary according to the nature and severity of the disease condition, the nature of the subject, and the potency of the composition. This amount can further depend upon the patient's height, weight, sex, age and medical history. A therapeutically effective dose or amount may
be sufficient to stimulate or augment the epithelial and/or endothelial wound healing response and, thus, induce or potentiate wound healing.

The term "pharmaceutically acceptable" refers to compounds and compositions which may be administered to mammals without undue toxicity. Suitable excipients include, but are not limited to, water, saline, Ringer's solution, dextrose solution, and solutions of ethanol, glucose, sucrose, dextran, mannose, mannitol, sorbitol, polyethylene glycol (PEG), phosphate, acetate, gelatin, collagen, CARBOPOL®, vegetable oils, white petrolatum, and the like, or a combination thereof. One may additionally include one or more suitable preservatives, stabilizers, antioxidants, antimicrobials, and buffering agents, for example, BHA, BHT, citric acid, ascorbic acid, tetracycline, and the like, and combinations thereof. In addition, various adjuvants commonly used in the art may be included. These and other such compounds are described, for example, in the literature, *e.g.,* in the Merck Index, Merck & Company, Rahway, N.J. Considerations for the inclusion of various components in pharmaceutical compositions are described, *e.g*., in Gilman et al. (Eds.) (2006); Goodman and Gilman's: The Pharmacological Basis of Therapeutics, 11th Ed., The McGraw-Hill Companies.

As used herein, the term "wound healing" refers to augmenting, improving, increasing, or inducing closure, healing, or repair of a wound. Wound healing is considered to be promoted, for example, if the time of healing a wound treated with a composition described herein compared to an untreated wound or a wound treated with a placebo substance is decreased by about 5%, about 10%, about 20%, about 25%, about 30%, about 40%, about 50%, about 75% or more. Conversely, the degree of scar formation can be used to ascertain whether wound healing is promoted. Wound healing, as described herein, also encompasses stimulating epidermal cells, causing short term fibrosis deposits, preventing re-opening of wounds, recruiting white blood cells to help growth factor and immune system activation (enzyme antibiotic effect), inducing greater re-growth of hair, reducing alopecia, enhancing epidermal restoration and integrity beyond that of the normal restorative process, or a combination thereof.

The wound can be an internal wound or an external wound found in any location of a mammal. A wound is a type of physical trauma where the integrity of the skin or tissue is disrupted as a result from *i.e.,* external force, bad health status, aging, exposure to sunlight, heat or chemical reaction or as a result from damage by internal physiological processes. If the outer layer of a tissue is damaged the wound is considered an open wound.

Wounds can also be caused by surgical procedures, such as open heart surgery, organ transplants, amputations, and implantations of prosthetics, such as joint and hip replacement, *etc.*

The wound can be an open wound or closed wound.

Open wounds refers to wounds in which the skin is broken. Open wounds include, for example, incisions (*i.e.,* wounds in which the skin is broken by, for instance, a cutting instrument (*e.g.,* knife, razor, *etc.*), lacerations (*i.e.,* wounds in which the skin is typically broken by a dull or blunt instrument), abrasions (*e.g.*, generally a superficial wound in which the topmost layers of the skin are scraped off), puncture wounds (typically caused by an object puncturing the skin, such as nail or needle), penetration wounds (*e.g*., caused by an object such as a knife), and gunshot wounds.

Closed wounds are typically wounds in which the skin is not broken. Closed wounds include for example contusions (or bruises) caused by a blunt force trauma that damages tissue under the skin, hematomas caused by damage to a blood vessel that in turn causes blood to collect under the skin, crush injury caused by a great or extreme amount of force applied over a long period of time, acute and chronic wounds.

Non-limitative examples of wounds are: a burn wound is the injury resulting from exposure to heat, electricity, radiation (for example, sunburn and laser surgery), or caustic chemicals, skin wounds due to aging or the environment, this includes for example splits, dry skin, roughness of the skin and the like, wounds due to external force damaging the tissue, ulcers (lesion on the surface of the skin or a mucous surface). Wounds in Diabetes Mellitus are typically foot injuries due to numbness caused by nerve damage (diabetic neuropathy) and low blood flow to the legs and feet. The most serious injury is a foot ulcer. Diabetic foot ulcers are at very high risk of becoming infected, and sometimes they cannot be healed. Non-healing foot ulcers are a frequent cause of amputation in people with diabetes, decubitus wounds, decubitus (bedsores), *i.e.,* lesions caused by unrelieved pressure to any part of the body, especially portions over bony or cartilaginous areas.

The pharmaceutical composition as described here above may be for
wound healing, stimulating epidermal cells, causing short term fibrosis deposits, preventing re-opening of wounds, recruiting white blood cells to help growth factor and immune system activation (enzyme antibiotic effect), inducing greater re-growth of hair, reducing alopecia, enhancing epidermal restoration and integrity beyond that of the normal restorative process, or a combination thereof.

Compositions described herein do not cause an allergic reaction, scarring, biological damage, burns, or a combination thereof.

In one embodiment, the composition is used for treating acute or chronic wounds.

Acute wounds are caused by external damage to intact skin and may be classified into different types, according to the object that caused the wound: for example, incisions or incised wounds, lacerations, abrasions and grazes, burns, puncture wounds caused by an object puncturing the skin, such as a nail or a needle, penetration wounds caused by an object such a knife entering the body, gunshot wounds caused by a bullet or similar projectile driving into or through the body. Acute wounds may also be closed wounds, such as contusions or bruises, hematoma, crushing injuries caused by a great or extreme amount of force applied over a long period of time. Other acute wounds are due to dermatologic diseases such as psoriasis, acne and eczema.

Chronic wounds are most frequently caused by endogenous mechanisms associated with a predisposing condition that ultimately compromises the integrity of dermal or epithelial tissue. Common chronic wounds are venous ulcers, which usually occur in the legs and mostly affect the elderly, diabetic ulcers which is another major cause of chronic wounds, pressure ulcers, which usually occur in people with conditions such as paralysis that inhibit movement of body parts that are commonly subjected to pressure such as the heels, shoulder blades and sacrum, corneal ulcers, most commonly caused by an infection with bacteria, viruses, fungi or amoebae, and digestive ulcers. All chronic wounds heal slowly and in an unpredictable manner.

Accordingly, the compositions described herein may be used for activating angiogenesis and, thereby, promote healing of wounds.

The compositions may be aqueous solutions, emulsions, creams, ointments, lotions, suspensions, gels, liposomal suspensions, and the like. Additional non-limiting examples of compositions for topical administration include, but are not limited to, a lotion, salve, gel, cream, balsam, tincture, cataplasm, elixir, paste, spray, collyrium, drops, suspension, dispersion, hydrogel, ointment, emulsion or powder. Other topical formulations include aerosols, bandages, dressing materials, alginate dressing and other wound dressings.

### Compositions

A composition for use as described herein can include one or more digestive enzymes. While not being bound by theory, it is believed that the digestive enzyme(s) in the composition can heal wounds, stimulate epidermal cells, cause short term fibrosis deposits, prevent re-opening of wounds, recruit white blood cells to help growth factor and immune system activation (enzyme antibiotic effect), induce greater re-growth of hair, reduce alopecia, enhancing epidermal restoration and integrity beyond that of the normal restorative process, or a combination thereof.

A digestive enzyme as described herein is an enzyme that can break down one or more components of food (*e.g*., proteins, fats, carbohydrates). The digestive enzymes can be animal-derived (*e.g*., pancreatic or other digestive-track enzymes), or plant-, fungal-, or microorganism-derived enzymes, or can be synthetically prepared. Many digestive enzymes are commercially available or can be isolated and purified from other sources by methods well known to those having ordinary skill in the art. Enzymatic activity of the enzymes can also be evaluated using standard assays.

The digestive enzymes can be used in any combination of type of enzyme and any combination of enzyme sources. In some embodiments, the digestive enzymes comprise enzymes selected from the group consisting of proteases, amylases, cellulases, sucrases, maltases, papain (*e.g.,* from papaya), bromelain (*e.g.,* from pineapple), hydrolases, and lipases. In some embodiments, the digestive enzymes comprise one or more pancreatic enzymes. In embodiments of the invention the composition comprises one or more proteases, one or more lipases, and one or more amylases. In some embodiments, the one or more proteases comprise chymotrypsin and trypsin. In some embodiments, a composition as described herein consists essentially of, or consists of, the digestive enzymes.

In certain embodiments, the composition can comprise at least one amylase, at least two proteases, and at least one lipase. In certain embodiments, the composition can further include one or more hydrolases, papain, bromelain, papaya, cellulases, pancreatin, sucrases, and maltases.

As indicated, the one or more digestive enzymes can be derived from an animal source. In some embodiments, the animal source is a pig, *e.g.,* a pig pancreas. Pig pancreatic enzyme extracts and formulations are known to those having ordinary skill in the art and are commercially available or can be prepared using known methods. For example, a pancreatic enzyme composition can be purchased from Scientific Protein Laboratories (designated PEC). A pancreatic enzyme composition, or any composition herein, can be adjusted to modify the amount of one or more digestive enzymes contained therein, *e.g.,* the lipase, amylase, or protease content, such as by production and/or processing methods or by the selective addition of exogenous enzymes, activators, or inhibitors to the composition.

Digestive enzymes to be used in the compositions and methods described herein include, for example, pancreatic enzymes. There are two types of pancreatic enzymes which have U.S.P. designations: pancreatin and pancrealipase. Pancreatin is a substance containing enzymes, principally amylase, lipase, and protease, obtained from the pancreas of the hog *Sus scrofa* Linne var. *domesticus* Gray (Fam. Suidae) or of the ox *Bos Taurus* Linne (Fam. Bocidae). Pancreatin contains, in each mg, not less than 25 U.S.P. units of amylase activity, not less than 2 U.S.P. units of lipase activity, and not less than 25 U.S.P. of protease activity. More information on Pancreatin is provided in Example 1 below. In contrast, pancrealipase U.S.P. refers to a cream-colored, amorphous powder, having a faint characteristic (meaty), but not offensive odor, which contains Lipase in an amount of not less than 24 U.S.P. Units/mg; Protease in an amount of not less than 100 U.S.P. Units/mg; and Amylase in an amount of not less than 100 U.S.P. Units/mg; with not more than 5% fat and not more than 5% loss on drying.

In certain circumstances, it may be desirable to have relatively higher activity of proteases than lipases. Thus, in some examples disclosed herein, a composition comprises at least one protease and at least one lipase, wherein the ratio of total proteases to total lipases (in U.S.P. units) ranges from about 1:1 to about 20:1 including about 1:1, about 2:1, about 3;1, about 4:1, about 5;1, about 6:1, about 7:1, about 8:1, about 9:1, about 10:1, about 11; 1, about 12;1, about 13;1, about 14:1, about 15:1, about 16;1, about 17:1, about 18:1, about 19:1 and about 20:1, long with all values in-between. The ratio (in U.S.P. units) of proteases to lipases may range from about 4:1 to about 10:1 including about 4:1, about 5:1, about 6:1, about 7:1, about 8:1, about 9:1, and about 10:1, along with all values in-between.

In certain circumstances it may be useful to modify the amount of a particular enzymatic activity in a given composition. The activity of the one or more digestive enzymes can be adjusted in a variety of ways known to the skilled artisan, *e.g.,* by increasing the amount of the particular enzyme, or by adjusting the components of the composition, *e.g.,* via the use of stabilizers, inhibitors, and activators. In some embodiments, a composition described herein includes one or more proteases having an activity of from about 0.05 to about 400 U.S.P. Units per mg of the composition, or any value there between (*e.g.,* about 0.1; about 0.2; about 0.25; about 0.5; about 1, about 2, about 5, about 10, about 15, about 20, about 25, about 30, about 35, about 40, about 45, about 50, about 55, about 60, about 65, about 75, 100, about 150, about 200, about 250, about 300, about 350 U.S.P. Units per mg). In some embodiments, a composition described herein includes one or more lipases having an activity of from about 0.005 to about 80 Units per mg of the composition, or any value there between (*e.g*., about 0.01, about 0.02, about 0.025, about 0.03, about 0.04, about 0.05, about 0.06, about 0.08, about 0.1, about 0.2, about 0.5, about 1, about 2, about 3, about 4, about 5, about 6, about 7, about 8, about 9, about 10, about 12, about 14, about 16, about 18, about 20, about 22, about 25, about 28, about 30, about 35, about 38, about 40, about 45, about 48, about 50, about 52, about 55, about 58, about 60, about 63, about 66, about 68, about 70, about 72, about 75, about 78, or about 80 U.S.P. Units per mg). In some embodiments, a composition described herein includes one or more amylases having an activity of from about 0.05 to about 500 U.S.P. Units per mg of the composition, or any value there between (*e.g.,* about 0.1; about 0.2; about 0.25; about 0.5; about 1, about 2, about 5, about 10, about 15, about 20, about 25, about 30, about 35, about 40, about 45, about 50, about 55, about 60, about 65, about 75, about 100, about 150, about 200, about 250, about 300, about 350, about 400 or about 450 U.S.P. Units per mg). In some embodiments, a composition described herein includes one or more proteases in the above activity range, one or more lipases in the above activity range, and one or more amylases in the above activity range. One example disclosed herein includes one or more proteases having an activity in the range of about 150-250 U.S.P. units/mg; one or more lipases having an activity in the range of about 20-40 U.S.P. units/mg; and one or more amylases having an activity in the range of about 200-300 U.S.P. units/mg.

In some embodiments, a composition can be formulated so as to stabilize the one or more digestive enzymes, *e.g.,* to preserve the enzymatic activity of the enzymes. Stabilization techniques can limit or prevent auto-degradation of the one or more enzymes in a composition and help maintain enzymatic activity, increase shelf-life, and aid in the tolerance of the activity of the compositions to changes in temperature, humidity, and storage conditions. In other applications, variations in excipients, pH, enzyme inhibitors, *etc.,* can be employed to aid in stabilizing the enzymes. Appropriate stabilization techniques will depend on the intended application for the composition, the form of the composition, the intended site of delivery/activity, and other factors, and can be determined by those in the art.

Certain useful enzyme activity stabilizers include compounds that provide a source of free calcium in a solution such as for example calcium salts; alkyl or branched alcohols such as for example ethanol and isopropyl alcohol; alkanolamines such as for example triethanolamine; acids, such as organic acids; and mixtures of petroleum distillates.

In certain embodiments, an enzyme activity stabilizer can be a composition selected from (1) compositions known to be effective in stabilizing enzymes in liquid aqueous solutions, including enzyme stabilizing compounds and systems, (2) selected "micelle inhibitors", and mixtures of (1) and (2). In some embodiments, the activity stabilizer is a suitable concentration of boron anions. In some cases, the activity stabilizer is solvated in a polyol and may be combined with enzyme stabilizing synergists or adjuvants forming an enzyme stabilizing system. Preferred "micelle inhibitors" include species known to modify as well as to inhibit micelle formation and may be selected from water miscible solvents such as C₁-C₆ alkanols, C₁-C₆ diols, C₂-C₂₄ alkylene glycol ethers, alkylene glycol alkyl ethers, and mixtures thereof. A highly preferred micelle inhibitor is di-(propylene glycol) methyl ether ("DPM") and analogues thereof which modify micelle formation.

One example of an "enzyme stabilizing system" is a boron compound (*e.g.,* boric acid) which in the past has been used alone or with selected other adjuvants and or synergists (*e.g.* polyfunctional amino compounds, antioxidants, *etc.)* to protect proteolytic and other enzymes in storage and in various products.

Other additives for inclusion in the compositions described herein can be determined by those having ordinary skill in the art, and will be based on a number of features, including intended application, *e.g.,* human vs. veterinary applications; desired release profile; desired pharmacokinetics; safety; stability; and physical characteristics (smell, color, taste, pour, aerosilization). Suitable formulation ingredients, excipients, binders, bulking agents, flavorants, colorants, *etc.,* can be determined and evaluated by methods known to those in the art.

Disclosed herein is a composition for wound healing comprising: from about 25 to 700,000 U.S.P. units protease, and 2 to 100,000 U.S.P. units lipase, and 25 to 400,000 U.S.P. units of amylase, in a base of white petrolatum.

In another embodiment, provided herein is a composition for wound healing comprising: 122,130 U.S.P. units protease, 17,110 U.S.P. units lipase, and 73,750 U.S.P. units amylase, in a base of 30 grams of white petrolatum.

In another embodiment, provided herein is a composition for wound healing comprising: 238,050U.S.P. units protease, 33,350 U.S.P. units lipase, and 143,750 U.S.P. units amylase, in a base of 30 grams of white petrolatum.

In another embodiment, provided herein is a composition for wound healing comprising: 459,540 U.S.P. units protease, 64,380 U.S.P. units lipase, and 277,500 U.S.P. units amylase, in a base of 30 grams of white petrolatum.

### Compositions for Human or Veterinary Use

Compositions described herein can be formulated as pharmaceutical compositions, e.g., can include a composition as described previously formulated with one or more pharmaceutically acceptable carriers or excipients. The pharmaceutical compositions are useful for wound healing in humans and other animals, such as mammals and birds.

Administration of the pharmaceutical compositions herein can be topical.

In the pharmaceutical compositions, effective concentrations of one or more digestive enzymes are mixed with a suitable pharmaceutical excipient or carrier. The concentrations of the digestive enzymes in the compositions are effective for delivery of an amount, upon administration, that is useful in wound healing and for stimulating epidermal cells, causes short term fibrosis deposits, preventing re-opening of wounds, recruiting white blood cells to help growth factor and immune system activation (enzyme antibiotic effect), inducing greater re-growth of hair, reducing alopecia, enhancing epidermal restoration and integrity beyond that of the normal restorative process, or a combination thereof. In one non-limiting example, a composition comprises proteases, lipases and amylases in a base of white petrolatum.

The digestive enzymes are included in the pharmaceutically acceptable carrier in an amount sufficient to exert a therapeutically useful effect in the absence of undesirable side effects on the patient treated. The therapeutically effective concentration may be determined empirically by testing the digestive enzymes in *in vitro* and *in vivo,* and then extrapolated therefrom for dosages for humans.

The concentration of digestive enzymes in the pharmaceutical composition will depend on absorption, inactivation and excretion rates of the enzymes, the physicochemical characteristics of the enzymes, the dosage schedule, the dosage form, and amount administered as well as other factors known to those of skill in the art.

The pharmaceutical composition may be administered at once, or may be divided into a number of smaller doses to be administered at intervals of time. It is understood that the precise dosage and duration of treatment is a function of the wound and may be determined empirically using known testing protocols or by extrapolation from in vivo or in vitro test data. It is to be noted that concentrations and dosage values may also vary with the severity of the wound. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions. In some embodiments, the compositions are provided in unit dosage forms suitable for single administration, or multi-dose administration, of a precise dose.

Upon mixing or addition of the digestive enzymes, the resulting mixture may be in a form suitable for topical administration. The form of the resulting mixture depends upon a number of factors, including the intended mode of administration and the solubility of the digestive enzymes in the selected carrier or vehicle.

The compositions can be administered either alone or more typically in combination with a conventional pharmaceutical carrier, excipient or the like. The term "excipient" is used herein to describe any ingredient other than the compound(s) (enzymes) used in the composition as described herein and known in the art.

Methods of preparing such dosage forms are known, or will be apparent, to those skilled in this art; for example, see Remington: The Science and Practice of Pharmacy, 21st Edition (Lippincott Williams & Wilkins. 2005).

Appropriate dosages for wound healing will depend on the patient (species, age, weight, health), the severity of the wound, the type of formulation and other factors known to those having ordinary skill in the art. It is to be noted that concentrations and dosage values may vary with the severity of the wound. It is to be further understood that for any particular patient, specific dosage regimens should be adjusted over time according to the individual need and the professional judgment of the person administering or supervising the administration of the compositions.

The pharmaceutical composition may comprise per dose:
amylases from about 10,000 to about 400,000U.S.P.units, including about 10,000, about 15,000, about 20,000, about 25,000, about 30,000, about 35,000, about 40,000, about 45,000, about 50,000, about 55,000, about 60,000, about 70,000, about 75,000, about 80,000, about 85,000, about 90,000, about 100,000, about 150,000, about 200,000, about 250,000, about 300,000, about 350,000 and about 400,000 U.S.P. units, along with all values in-between, proteases from about 10,000 to about 700,000 U.S.P. units, including about 10,000, about 15,000, about 20,000, about 25,000, about 30,000, about 35,000, about 40,000, about 45,000, about 50,000, about 55,000, about 60,000, about 65,000, about 70,000, about 75,000, about 80,000, about 85,000, about 90,000, about 95,000, about 100,000, about 105,000, about 110,000, about 115,000, about 120,000, about 125,000, about 130,000, about 135,000, about 140,000, about 145,000, about 150,000, about 155,000, about 160,000, about 165,000, about 170,000, about 200,000, about 250,000, about 300,000, about 350,000, about 400,000, about 450,000, about 500,000, about 550,000, about 600,000, about 650,000 and about 700,000 U.S.P. units along with all values in-between, and lipases from about 4,000 to about 100,000 U.S.P. units, including 4,000, 5,000, 10,000, 15,000, 20,000, 25,000, 30,000, 35,000, 45,000, 55,000, 60,000, 70,000, 80,000, 90,000, 95,000 and 100,000 U.S.P. units along with all values in-between A pharmaceutical composition can include one or more of: chymotrypsin from about 2 to about 20 mg including about 2.0, about 2.5, about 3.0, about 3.5, about 4.0, about 4.5, about 5.0, about 6, about 7, about 8, about 9, about 10, about 11, about 12, about 13, about 14, about 15, about 16, about 17, about 18, about 19 and about 20 mg along with all values in-between; trypsin from about 30 to about 100 mg including about 30, about 35, about 40, about 45, about 50, about 65, about 70, about 75, about 80, about 85, about 90, about 95 and about 100 mg, including all values in between; papain from about 3,000 to about 10,000 U.S.P. units including about 3,000, about 4,000, about 5,000, about 6,000, about 7,000, about 8,000, about 9,000, and about 10,000 U.S.P., along with all values in between; and papaya from about 30 to about 60 mg, including about 30, about 35, about 40, about 45, about 50, about 55, and about 60 mg, along with all values in between.

Additional information on particular dosage forms of the compositions is provided below.

Topical mixtures can be prepared as described for local administration. The resulting mixture may be a solution, suspension, emulsions or the like and are formulated as creams, gels, ointments, emulsions, powders, solutions, elixirs, lotions, suspensions, tinctures, pastes, foams, aerosols, irrigations, sprays, suppositories, bandages, dermal patches or any other formulations suitable for topical administration.

The digestive enzymes may be formulated for topical application, such as for topical application to the skin and mucous membranes, in the form of gels, creams, and lotions. Topical administration is contemplated for transdermal delivery and also for administration to the eyes or mucosa.

Powders can be formed with the aid of any suitable powder base, *e.g.,* talc, lactose, starch, and the like. Solutions can be formulated with an aqueous or non-aqueous base, and can also include one or more dispersing agents, suspending agents, solubilizing agents, and the like. Topical gels are prepared using polymers having a molecular weight and level of concentration effective to form a viscous solution or colloidal gel of an aqueous or non-aqueous solution or suspension of digestive enzymes. Polymers from which topical gels may be prepared include polyphosphoesters, polyethylene glycols, high molecular weight poly(lactic) acids, hydroxypropyl celluloses, chitosan, polystyrene sulfonates, and the like.

Ointments, creams and lotions are formulated, for example, with an aqueous or oily base and addition of a suitable thickening agent, gelling agent, stabilizing agent, emulsifying agent, dispersing agent, suspending agent, or consistency regulating agent, and the like. Bases include water, an alcohol, or an oil, such as liquid paraffin, mineral oil, or a vegetable oil, such as peanut or castor oil. Thickening agents that can be used according to the nature of the base include soft paraffin, aluminum stearate, cetostearyl alcohol, propylene glycol, polyethylene glycols, polyphosphoesters, poly(lactic acids), hydroxyethyl celluloses, hydroxypropyl celluloses, cellulose gums, acrylate polymers, hydrophilic gelling agents, chitosan, polystyrene sulfonate, petrolatum, woolfat, hydrogenated lanolin, beeswax, and the like.

The ointments, pastes, creams, gels, and lotions can also contain excipients, such as animal and vegetable fats, oils, waxes, paraffins, starch, tragacanth, cellulose derivatives, polyethylene glycols, silicones, bentonites, silicic acid, talc, zinc oxide, and mixtures thereof. Powders and sprays can also contain excipients such as silicic acid, aluminum hydroxide, calcium silicates and polyamide powder, or mixtures of these substances. Solutions, suspensions or dispersions can be converted into aerosols or sprays by any of the known means routinely used for making aerosols for topical application. In general, such methods comprise pressurizing or providing a means of pressurizing a container of a solution, suspension or dispersion, usually with an inert carrier gas, and passing the pressured gas through a small orifice. Sprays and aerosols can also contain customary propellants, *e.g.,* chlorofluorohydrocarbons or volatile unsubstituted hydrocarbons, such as butane and propane.

Excipients for use in the compositions described herein include any excipient for use in a composition that may be applied for therapeutic purposes. One or more excipients may comprise, for example, water, saline, Ringer's solution, dextrose, ethanol, glucose, sucrose, dextran, mannose, mannitol, sorbitol, polyethylene glycol (PEG), phosphate, acetate, gelatin, collagen, CARBOPOL®, vegetable oils, white petrolatum or a combination thereof.

Additional excipients include, but are not limited to, compounds that promote skin absorption, such as dimethyl sulfoxide (DMSO), partial glycerides of fatty acids, and the like, present at levels up to about 10 wt % of the total formula weight. Examples of partial fatty acid glycerides include, but are not limited to IMWTTOR® 742 and IMWITOR 308 available from SASOL North America, Inc., of Houston, Texas. The topical formulations may also optionally include inactive ingredients to improve cosmetic acceptability, including but not limited to, humectants, surfactants, fragrances, coloring agents, emollients, fillers, and the like.

Compositions may also, in some instances, further comprise one or more suitable preservatives, stabilizers, antioxidants, antimicrobials, buffering agents, or a combination thereof.

Suitable preservatives include, but are not limited to, acids, alcohols, glycols, parabens, quaternary-nitrogen containing compounds, isothiazolinones, aldehyde-releasing compounds and halogenated compounds. In one embodiment, preservatives for use herein include, but are not limited to, imidazolidinyl urea, diazolidinyl urea, phenoxyethanol, methylparaben, ethylparaben, propylparaben, or a combination thereof. Additional examples of preservatives useful for the purpose of the present disclosure can be found in Steinberg, D. "Frequency of Use of Preservatives 2007". Cosmet. Toilet. 117, 41-44 (2002) and, "Preservative Encyclopedia" Cosmet. Toilet. 117, 80-96 (2002).

A wide variety of acids, bases, and buffers may be utilized to adjust and/or maintain the pH of the compositions useful in the present methods. Examples of materials useful for adjusting and/or maintaining the pH include, without limitation, phosphate, citrate, and other organic acids; ammonia, sodium carbonate, sodium hydroxide, triethanolamine, hydrochloric acid, phosphoric acid, sodium hydrogen phosphate, sodium dihydrogen phosphate, citric acid, and the like.

Suitable antioxidants for use herein include, but are not limited to, ascorbic acid and methionine.

These ingredients are present in a safe and effective amount in a topical cosmetically acceptable carrier, which can be of a variety of different forms.

The pharmaceutically-acceptable topical carrier, in total, typically comprises from about 0.1% to about 95% by weight of the composition of step one above, from about 70% to about 91%, or from about 80% to about 90%.

Suitable surfactants for use herein include, but are not limited to, TWEEN® (*e.g*., TWEEN® 20 or TWEEN® 80), polysorbate (*e.g*., polysorbate 20 or polysorbate 80), PLURONICS® (*e.g*., PLUROMC® F68), polyethylene glycol (PEG) and the like.

The topical compositions may be administered directly by the dusting of a powder, spraying of an aerosol or by spreading a film of an ointment, cream, lotion, solution or gel to the desired area of the skin using the fingertips of the patient or a healthcare provider or other conventional application such as a swab or wipe. The product may be first applied to the skin and spread with the fingertips or an applicator or applied to the fingertips and spread over the skin. The compositions may also optionally first be coated on the surface of a topical applicator, such as a bandage, swab, moist woven or non-woven wipe and the like, which is then applied to the portion of the skin to receive the composition.

The topical compositions may be prepared with base formulations that are essentially conventional to one of ordinary skill in the art with respect to the ingredients employed, quantities thereof, and methods of preparation, all of which require no further description. Topical compositions may be prepared as a cream or lotion based on an emulsion formulation possessing heretofore unrecognized wound healing activity, in addition to good skin compatibility.

Compositions for use described herein are not limited to topical cream or lotion formulations. Topical formulations may also be formulated as powders, sprays, lotions, creams, aqueous and non-aqueous solutions, liquids, oils, gels, ointments, pastes, unguents, emulsions and suspensions; such compositions will contain an amount of digestive enzymes, and optionally one or more other wound healing agents, in a total concentration of between about 0.125% and about 25% by weight or more, recognizing again that optimal dosages may differ only by 0.05% by weight, so that representative cream and lotion embodiments will include every 0.05% by weight concentration increment within this range.

Topical compositions may be used to treat skin infections and wound infections such as surface wounds and penetrating wounds. Wounds suitable for treatment include acute and chronic wounds, such as, for example, wounds in skin abrasions, skin or surface cuts, decubiti, burns and surgical wounds.

Also of interest herein are also lyophilized powders, which can be reconstituted for administration as solutions, emulsions and other mixtures. They may also be reconstituted and formulated as gels.

The sterile, lyophilized powder is prepared by dissolving digestive enzymes as provided herein in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at, in one embodiment, about neutral pH. Subsequent sterile filtration of the solution followed by lyophilization under standard conditions known to those of skill in the art provides the desired formulation. In one embodiment, the resulting solution will be apportioned into vials for lyophilization. Each vial will contain a single dosage or multiple dosages of the digestive enzymes. The lyophilized powder can be stored under appropriate conditions, such as at about 4° C to room temperature.

For reconstitution, the lyophilized powder is added to sterile water or other suitable carrier. The precise amount depends upon the selected digestive enzymes. Such amount can be empirically determined.

### Combination therapy

Compositions described herein may further include one or more other wound healing agents. Alternatively, compositions described herein may be used in combination with at one or more other wound healing agents.

Such other wound healing agents include, but are not limited to, growth factors, cytokines, enzymes, and extra-cellular matrix components. For example, collagenase treatment of the sub-endothelial extracellular matrix in combination with the enzymes may synergistically accelerate endothelial migration and proliferation to a level greater than the inductive influence of collagenase treatment in the absence of the enzymes.

Agents that effect wound repair can also be included in such a composition to augment the wound healing process. Such agents include members of the family of growth factors, such as insulin-like growth factor (IGF-1), platelet derived growth factor (PDGF), epidermal growth factor (EGF), transforming growth factor beta (TGF-β), basic fibroblast growth factor (bFGF), thymosin α1 (Tα1) and vascular endothelial growth factor (VEGF). In one embodiment, the agent is transforming growth factor beta (TGF-β) or other members of the TGF-β superfamily. In another embodiment, a composition further comprises a haemostatic substance, a growth factor, an anti-infective substance, an analgesic substance, an anti-inflammatory substance or a combination thereof.

### Medical uses

Pharmaceutical compositions described herein can be used to treat any patient having an acute or chronic wound. The pharmaceutical compositions can be in any appropriate dosage form (*i.e.,* single or multi-dosage), as described previously.

Pharmaceutical compositions described herein may reduce scarring and promote wound healing in patients having infected wounds. Additionally, pharmaceutical compositions described herein may be used to stimulate epidermal cells, cause short term fibrosis deposits, prevent re-opening of wounds, recruit white blood cells to help growth factor and immune system activation (enzyme antibiotic effect), induce greater re-growth of hair, reduce alopecia, enhancing epidermal restoration and integrity beyond that of the normal restorative process, or a combination thereof. In other embodiments, pharmaceutical compositions described herein can be used on their own, and/or in combination with other therapeutic wound healing agents.

In one embodiment, scarring is reduced by at least about 2-fold, about 3-fold, about 4-fold, about 5-fold, about 7.5-fold, about 10-fold, about 15-fold, about 20-fold, about 25-fold or more compared a subject treated with a placebo. In another embodiment, scarring is reduced by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

In one embodiment, scarring is reduced by at least about 2-fold, about 3-fold, about 4-fold, about 5-fold, about 7.5-fold, about 10-fold, about 15-fold, about 20-fold, about 25-fold or more compared to a subject not receiving treatment with a composition described herein. In another embodiment, scarring is reduced by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

In one embodiment, wound healing is increased by at least about 2-fold, about 3-fold, about 4-fold, about 5-fold, about 7.5-fold, about 10-fold, about 15-fold, about 20-fold, about 25-fold or more compared a subject treated with a placebo. In another embodiment, wound healing is increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

In one embodiment, wound healing is increased by at least about 2-fold, about 3-fold, about 4-fold, about 5-fold, about 7.5-fold, about 10-fold, about 15-fold, about 20-fold, about 25-fold or more compared to a subject not receiving treatment with a composition described herein. In another embodiment, wound healing is increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

In one embodiment, epithelial cells are stimulated by at least about 2-fold, about 3-fold, about 4-fold, about 5-fold, about 7.5-fold, about 10-fold, about 15-fold, about 20-fold, about 25-fold or more compared a subject treated with a placebo. In another embodiment, epithelial cells are stimulated by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

In one embodiment, epithelial cells are stimulated by at least about 2-fold, about 3-fold, about 4-fold, about 5-fold, about 7.5-fold, about 10-fold, about 15-fold, about 20-fold, about 25-fold or more compared to a subject not receiving treatment with a composition described herein. In another embodiment, epithelial cells are stimulated by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

In one embodiment, short term fibrosis deposits are increased by at least about 2-fold, about 3-fold, about 4-fold, about 5-fold, about 7.5-fold, about 10-fold, about 15-fold, about 20-fold, about 25-fold or more compared a subject treated with a placebo. In another embodiment, short term fibrosis deposits are increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

In one embodiment, short term fibrosis deposits are increased by at least about 2-fold, about 3-fold, about 4-fold, about 5-fold, about 7.5-fold, about 10-fold, about 15-fold, about 20-fold, about 25-fold or more compared to a subject not receiving treatment with a composition described herein. In another embodiment, short term fibrosis deposits are increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

In one embodiment, re-growth of hair is increased by at least about 2-fold, about 3-fold, about 4-fold, about 5-fold, about 7.5-fold, about 10-fold, about 15-fold, about 20-fold, about 25-fold or more compared a subject treated with a placebo. In another embodiment, re-growth of hair is increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

In one embodiment, re-growth of hair is increased by at least about 2-fold, about 3-fold, about 4-fold, about 5-fold, about 7.5-fold, about 10-fold, about 15-fold, about 20-fold, about 25-fold or more compared to a subject not receiving treatment with a composition described herein. In another embodiment, re-growth of hair is increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

In one embodiment, alopecia is reduced by at least about 2-fold, about 3-fold, about 4-fold, about 5-fold, about 7.5-fold, about 10-fold, about 15-fold, about 20-fold, about 25-fold or more compared a subject treated with a placebo. In another embodiment, alopecia is reduced by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

In one embodiment, alopecia is reduced by at least about 2-fold, about 3-fold, about 4-fold, about 5-fold, about 7.5-fold, about 10-fold, about 15-fold, about 20-fold, about 25-fold or more compared to a subject not receiving treatment with a composition described herein. In another embodiment, alopecia is reduced by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

In one embodiment, white blood cell recruitment is increased by at least about 2-fold, about 3-fold, about 4-fold, about 5-fold, about 7.5-fold, about 10-fold, about 15-fold, about 20-fold, about 25-fold or more compared a subject treated with a placebo. In another embodiment, white blood cell recruitment is increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared a subject treated with a placebo.

In one embodiment, white blood cell recruitment is increased by at least about 2-fold, about 3-fold, about 4-fold, about 5-fold, about 7.5-fold, about 10-fold, about 15-fold, about 20-fold, about 25-fold or more compared to a subject not receiving treatment with a composition described herein. In another embodiment, white blood cell recruitment is increased by at least about 2%, about 3%, about 4%, about 5%, about 7.5%, about 10%, about 15%, about 20%, about 25%, about 30%, about 35%, about 40%, about 45%, about 50%, about 55%, about 60%, about 65%, about 70%, about 75%, or more compared to a subject not receiving treatment with a composition described herein.

Compositions described herein can be evaluated for a variety of activities by methods known to those having ordinary skill in the art. For example, enzymatic activities can be evaluated using standard enzyme assays. Other assays are described in the Examples below.

### EXAMPLES

In order that those in the art may be better able to practice the compositions and methods described herein, the following examples are provided for illustration purposes.

The following study was conducted to determine the toxicity and toxicokinetic profile of the test article, CM-wh001, in low, middle and high dose concentrations of pancreatic enzyme complex in a base of white petrolatum, ***etc.,*** following a topical application to each side of the rabbit's dorsum (with the left side abraded and the right side unabraded) for 5 days followed by an observation period to allow for an assessment of healing and reversibility of any changes. Three (3) animals (1001A, 1002A and 1003A) were sent to necropsy 2 days following the last treatment. The remaining 3 rabbits (1004A, 1005A and 1006A) were sent to necropsy seven (7) days following the last treatment. Each test site was evaluated.

The study followed ITR Canada's Standard Operating Procedures (SOPs).

Topical administration was chosen as the route of administration because it is the human therapeutic route for treatment of wounds and to assess healing thereof.

Rabbits were selected because they are the non-rodent species recommended by various regulatory authorities for this type of study and for which background data are available.

| | |
|---|---|
| Species: | Rabbit (*Oryctolagus cuniculus)* |
| Strain: | New Zealand White, Crl: KBL (NZW) BR |
| Source: | Charles River Canada Inc., 188 rue Lasalle, St. Constant, Quebec, Canada |
| Total Animal No. in Study: | 6 males |
| Body Weight Range: | 2.8 to 3.1 kg |
| Age Range at Start: | Approximately 4 months |
| Acclimation Period: | 34 days |

The protocol for this study was reviewed and assessed by the Animal Care Committee (ACC) of ITR. ACC acceptance of the protocol was maintained on file at ITR. All animals used on this study were cared for in accordance with the principles outlined in the current "Guide to the Care and Use of Experimental Animals" as published by the Canadian Council on Animal Care and the "Guide for the Care and Use of Laboratory Animals", an NIH publication. The study described in this report did not unnecessarily duplicate previous experiments. On arrival at ITR, all animals were weighed and subjected to a detailed physical examination by a technician designated by the clinical veterinarian. The health status data is not reported but retained in the study file. Each animal was housed individually in rabbit stainless steel wire mesh-bottom cage equipped with an automatic watering system supplemented by water bottles as appropriate. The cage door locks were appropriately secured with a clip as necessary. After randomization, each cage was clearly labeled with a color-coded cage card indicating the study, group and animal numbers, and sex. Each animal was uniquely identified on the inside of the right ear by a permanent marker (renewed as necessary) following arrival. The animal room environment was controlled (targeted ranges: temperature 18.5 ± 3°C, relative humidity 50 ± 20%, 12 hours light, 12 hours dark and a minimum of 10 air changes per hour). Temperature and relative humidity was monitored continuously and records were maintained at ITR. A standard certified commercial rabbit chow (Teklad Global High Fiber Rabbit Diet #2031) was available to the animals daily (60 g on the first day after arrival, 120 g on the second day after arrival, 180 g on the third day after arrival and 200 g thereafter), except on the day of arrival. During the pre-treatment and treatment periods the animals were given regular commercial rabbit pellets mixed with baby carrots and/or alfalfa for appetite enhancement.

Municipal tap water (which was purified by reverse osmosis, ultraviolet light and further filtered with a 0.2 µm filter) was provided to the animals *ad libitum* except during designated procedures such as removal from the home cage for dosing or observation. Periodic analyses of municipal tap water (collected by the city) and reverse osmosis water from the animal rooms (collected by ITR) are performed by Exova Canada, Pointe-Claire, Quebec, Canada and the results are retained on file at ITR. It is considered that there were no known contaminants in the diet and water that would have interfered with the assessment of the objectives of the study. During the study, the animals were offered non-dietary items (*i.e.,* NYLABONE®) as part of the ITR environmental enrichment program.

An acclimation period of 34 days was allowed between receipt of the animals and the start of treatment to accustom the rabbits to the room environment. All rabbits were acclimated to the experimental procedures (*i.e.,* handling) for a minimum of 3 consecutive days, prior to the start of treatment.

An appropriate area on the dorsum of the rabbit (approximately 14 cm x 20 cm) was clipped free of hair close to the skin prior to selection of suitable study animals. This area was re-clipped on the day before start of treatment. Each dermal test site was an area of approximately 6 cm² (2 cm x 3 cm) sited on an area of skin free from active hair growth and pre existing damage and as high up the dorsum as possible to make it difficult for the animals to ingest the test material. The selected dosing area was marked at each corner of the site by a dot of non toxic indelible ink to facilitate observation in-life and identification at necropsy.

The left flank only was abraded on day 1 (*i.e.,* prior to the first treatment). Each of the abraded test sites was treated with a topical anesthetic at least 15 minutes but not more than 30 minutes prior to abrasion. Abrasion was achieved using a sterile scalpel blade which was gently drawn across the test site to create a cross hatch pattern. The intention of this was not to cause a deep wound but to slightly cut the epidermis such that some clear tissue fluid was allowed to escape. Occasionally a small amount of blood was released but this was not general. Care was taken to ensure that all sites and animals were similarly abraded by one person.

The dosing area was re-marked and clipped as necessary to allow Draize scoring only. All possible care was taken when clipping the dermal test site to ensure that no damage was caused to the skin.

### Protocol

Test (low, mid and high doses) and control (no active) formulations were administered daily by topical application with the tip of a gloved finger to 6 male rabbits for 5 consecutive days. The compounds were applied to each side of the rabbit's dorsum with the left side abraded and the right side unabraded as shown in Table 1. The weight/volume administered to each animal was 0.2 gm (on each site).

### Test formulation:

CM-wh001 is a high protease enzyme concentrate comprised of proteases, lipases and amylases in a white petrolatum base, developed for the treatment of wounds. Dilutions of the test formulation were chosen on the basis that administration thereof would not result in any caustic effect. Ad hoc testing on human intact skin (n=4) at these dilutions showed no evidence of irritation.

Low dose: Contained 122,130 U.S.P. units protease, 17,110 U.S.P. units lipase, and 73,750 U.S.P. units amylase, in 30 grams of white petrolatum.

Mid dose: Contained 238,050 U.S.P. units protease, 33,350 U.S.P. units lipase, and 143,750 U.S.P. units amylase, in 30 grams of white petrolatum.

High dose: Contained 459,540 U.S.P. units protease, 64,380 U.S.P. units lipase, and 277,500 U.S.P. units, amylase, in 30 grams of white petrolatum.

### Control formulation:

White petrolatum (colorless ointment)

### Administration schedule:

- **Day 0:** weight measurement, shave hair and detailed clinical examination (DCE);
- **Day 1:** abrade skin and apply first treatment. Dermal changes assessed and recorded at 1 hours and 4 hours post dose;
- **Day 2:** dermal changes observed and apply treatment;
- **Day 3:** dermal changes observed and apply treatment;
- **Day 4:** dermal changes observed and apply treatment;
- **Day 5:** dermal changes observed and last treatment;
- **Day 6:** dermal changes observed;
- **Day 7:** dermal changes observed;
- **Day 8:** dermal changes observed, detailed clinical examination, weight measurement and necropsy of animal nos. 1001A, 1002A and 1003A;
- **Day 9:** dermal changes observed;
- **Day 10:** dermal changes observed;
- **Day 11:** dermal changes observed;
- **Day 12:** dermal changes observed; and
- **Day 13:** dermal changes observed, detailed clinical examination, weight measurement and necropsy of animal nos. 1004A, 1005A and 1006A.

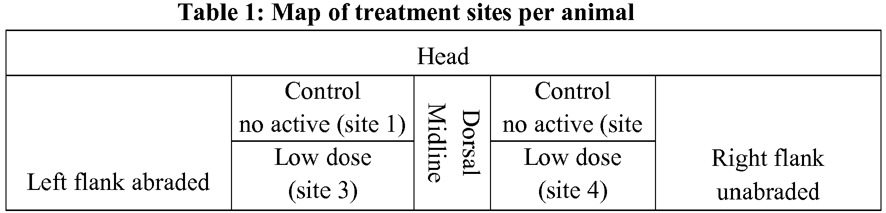

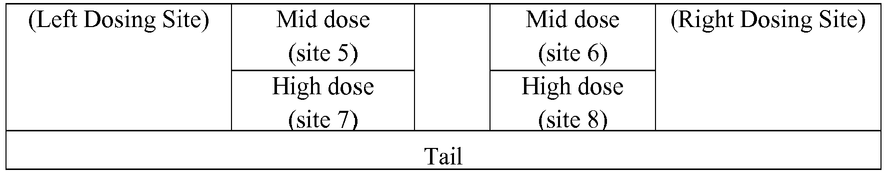

Briefly, the test and control placebo articles were administered by dermal application to the clipped dorsum for 5 consecutive days (*i.e.,* days 1, 2, 3, 4, and 5). The appropriate volume of test or placebo article was applied to the dermal test site and spread uniformly over the surface of the skin. This was achieved using a gloved finger and it was ensured that excessive residue did not remain on the glove. Gloves were changed between each dose and animal. The same dermal test site was used for all doses on each dosing day.

The dermal test areas were covered with a layer of gauze after each treatment and held in place by an Elastoplast-type bandage. Every attempt was made to ensure that dosing was performed within the working day so that the normal cycles of light and dark in the animal room were maintained. Mortality checks were performed once a day during all phases of the study. Cage-side clinical signs (*e.g.,* ill health, behavioral changes, *etc.)* were recorded once daily during the acclimation period and once daily each morning during the treatment and observation periods. Animals whose health status was judged to warrant additional evaluation were examined by a Clinical Veterinarian or the Associate Clinical Veterinarian.

A DCE of each rabbit was performed once pretreatment, once during the treatment, and on days 8 and 13 prior to necropsy. Animals 1001A, 1002A and 1003A were observed during treatment and for 2 days after cessation of treatment (*i.e.,* day 8). Animals 1004A, 1005A and 1006A were observed during treatment and for 7 days after cessation of treatment (*i.e.,* day 13).

Dermal changes were assessed and recorded twice at 1 and 4 hours post dose on day 1 and then daily prior to each subsequent dose. After cessation of treatment, dermal changes were assessed and recorded prior to necropsy for all six animals on days 6, 7, 8 for animals 1001A, 1002A and 1003A and then daily (from day 9 until day 13) for animals 1004A, 1005A and 1006A. During the study, animals were monitored for possible mortality, clinical signs (*e.g*., illness and change of behavior) and Draize scoring of the dermal test sites.

Dermal observations were recorded using the following modified Draize scoring scheme:

| **Erythema/Eschar Formation** | **Score** |
|---|---|
| No erythema | **0** |
| Very slight erythema (barely perceptible) | **1** |
| Well-defined erythema (pale red in color | **2** |
| Moderate to severe erythema (definite red in color) | **3** |
| Severe erythema (beet or crimson red in color) to slight eschar formation (injuries in depth) | **4** |

| **Edema Formation** | **Score** |
|---|---|
| No edema | **0** |
| Very slight edema (barely perceptible) | **1** |
| Slight edema (edges of area well defined by definite raising) | **2** |
| Moderate edema (area well-defined and raised approximately 1 mm) | **3** |
| Severe edema (raised more than 1 mm and extending beyond the area of exposure) | **4** |

Assessment of healing was performed with reference to the control site during the Draize scoring. Healing was assessed using the following scale:

| **Healing assessment** | **Score** |
|---|---|
| No healing present | **0** |
| Slight healing present | **1** |
| Moderate healing present | **2** |
| Complete healing present | **3** |

Evaluation of the changes at the dermal test site was not confined to the scoring system noted above. In addition, particular attention was paid to the following changes:

| **Dermal Change** | **Description of Dermal Change/Comment** |
|---|---|
| Atonia | Decrease in normal elasticity |
| Desquamation | Scaling/flaking of the epidermis |
| Fissuring | Cracks in the skin |
| Scab/crust formation | It should be noted whether the crust appears to be due to leaking body fluid or residue of test formulation. |
| Erosion/ulceration | As per normal use |
| Scarring | New skin formation (shiny in appearance but not residue of test article) |
| Alopecia | Hair loss/lack of re-growth |
| Self-inflicted injury | Scratches/abrasions (not reported) |

Body weights were recorded for all animals once prior to group assignment and once during pre-treatment. Body weights were recorded for all animals up to 1 day prior to dosing and at termination (prior to necropsy). Rabbits were euthanized by intravenous overdose of Sodium Pentobarbital, followed by exsanguinations by transsection of major blood vessels. For each rabbit, necropsy included excision of the dermal test sites. The dermal test sites and animal IDs were retained in neutral buffered 10% formalin. Histopathological examination was performed on all the test sites from all animals.

No animals died as a result of the treatment, nor did the animals exhibit any signs of illness or abnormal behavior during, or after, treatment. Administration of CM-wh001 did not have any adverse effects on the body weight of the animals in this study.

### HISTOPATHOLOGY PROCEDURES

### Slide Preparation

Sections of the dermal test sites were prepared for microscopic examination by embedding in paraffin wax, sectioning and staining with hematoxylin and eosin using conventional methods.

Histological processing was conducted for all animals.

### Histopathological Examination

Histopathological examination was performed on all test sites from all animals.

### DATA CAPTURE

The following computerized systems were used during the conduct of this study: (1) In-life data collection (PROVAMTIS®) and (2) Post-life data collection (PROVANTIS®).

### DATA EVALUATION AND STATISTICS

Numeric and non-numeric data obtained during the study were reported only as individual values as appropriate and no statistical analysis were performed.

### STANDARD OPERATING PROCEDURES

All procedures were performed in accordance with the ITR Standard Operating Procedures and these have been kept on file at ITR. Deviations to the ITR Standard Operating Procedures were documented in the raw data.

### RESULTS

### Clinical Signs (Tables 2 -4)

There were no clinical signs (*e.g.,* ill health, behavioral changes, *etc.*) that could be attributed to the topical administration of the test article, CM-wh001.

Dosing sites were as follows:

| | |
|---|---|
| Dosing Sites 1 and 2 | Control |
| Dosing Sites 3 and 4 | Low Dose CM-wh001 |
| Dosing Sites 5 and 6 | Mid Dose CM-wh001 |
| Dosing Sites 7 and 8 | High Dose CM-wh001 |

**Table 2: CAGE SIDE OBSERVATIONS (PRETREATMENT PERIOD)**

| Day numbers relative to Start Date | | | | | | | |
|---|---|---|---|---|---|---|---|
| | | - | - | - | - | - | - |

| Animal | Clinical Sign | 6 | 5 | 4 | 3 | 2 | 1 |
|---|---|---|---|---|---|---|---|
| 1001A | No Abnormalities Detected | X | X | X | X | X | X |
| 1002A | No Abnormalities Detected | X | X | X | X | X | X |
| 1003A | No Abnormalities Detected | X | X | X | X | X | X |
| 1004A | No Abnormalities Detected | X | X | X | X | X | X |
| 1005A | No Abnormalities Detected | X | X | X | X | X | X |
| 1006A | No Abnormalities Detected | X | X | X | X | X | X |

| | | | | | | | |
|---|---|---|---|---|---|---|---|
| Severity Codes: X = Abnormalities were not present | | | | | | | |

**Table 3: CAGE SIDE OBSERVATIONS (TREATMENT PERIOD) Day numbers relative to Start Date**

| | | | | | | | | | | | | |
|---|---|---|---|---|---|---|---|---|---|---|---|---|
| Animal | Clinical Sign | 2 | 3 | 4 | 5 | 7 | 8 | 9 | 10 | 11 | 12 | 13 |
| 1001A | No Abnormalities Detected Scheduled Euthanasia | X | X | X | X | X | · | · | · | · | · | · |
| | | · | · | · | · | · | X | · | · | · | · | · |
| 1002A | No Abnormalities Detected | X | X | X | X | X | · | · | · | · | · | · |
| | Scheduled Euthanasia | · | · | · | · | · | X | · | · | · | · | · |
| 1003A | No Abnormalities Detected | X | X | X | X | X | · | · | · | · | · | · |
| | Scheduled Euthanasia | · | · | · | · | · | X | · | · | · | · | · |
| 1004A | No Abnormalities Detected | X | X | X | X | X | X | X | X | X | X | · |
| | Scheduled Euthanasia | · | · | · | · | · | · | · | · | · | · | X |
| 1005A | No Abnormalities Detected | X | X | X | X | X | X | X | X | X | X | · |
| | Scheduled Euthanasia | · | · | · | · | · | · | · | · | · | · | X |
| 1006A | No Abnormalities Detected | X | X | X | X | X | X | X | X | X | X | · |
| | Scheduled Euthanasia | · | · | · | · | · | · | · | · | · | · | X |

| | | | | | | | | | | | | |
|---|---|---|---|---|---|---|---|---|---|---|---|---|
| Severity Codes: X = Abnormalities were not present | | | | | | | | | | | | |

**Table 4 (Detailed Clinical Exination) Day numbers relative to Start Date**

| **Animal** | **Clinical Sign** | 7 | **1** | **6** | **8** | **13** |
|---|---|---|---|---|---|---|
| 1001A | No Abnormalities Detected | X | X | X | X | · |
| | Scheduled Euthanasia | · | · | · | X | · |
| 1002A | No Abnormalities Detected | X | X | X | X | · |
| | Scheduled Euthanasia | · | · | · | X | · |
| 1003A | No Abnormalities Detected | X | X | X | X | · |
| | Scheduled Euthanasia | · | · | · | X | · |
| 1004A | No Abnormalities Detected | X | X | X | · | X |
| | Scheduled Euthanasia | · | · | · | · | X |
| 1005A | No Abnormalities Detected | X | X | X | · | X |
| | Scheduled Euthanasia | · | · | · | · | X |
| 1006A | No Abnormalities Detected | X | X | X | · | X |
| | Scheduled Euthanasia | · | · | · | · | X |

| | | | | | | |
|---|---|---|---|---|---|---|
| Severity Codes: X = Abnormalities were not present | | | | | | |

### Body Weight (Table 5)

There was no effect on the body weight of the animals subsequent to the topical administration of three concentrations of CM-wh001.

**Table 5: BODY WEIGHTS (INDIVIDUAL VALUES, MEAN AND SD)**

| Animal | Bodyweight (kg): Day(s) Relative to Start Date | | |
|---|---|---|---|
| | -1 | 8 | 13 |
| 1001A | 3.0 | 3.0 | - |
| 1002A | 2.9 | 3.0 | - |
| 1003A | 3.0 | 3.1 | - |
| 1004A | 2.8 | - | 2.9 |
| 1005A | 3.0 | - | 3.1 |
| 1006A | 3.1 | - | 3.1 |

### Dermal Observations

Dermal changes were assessed using the evaluations listed above using the Draize scoring.

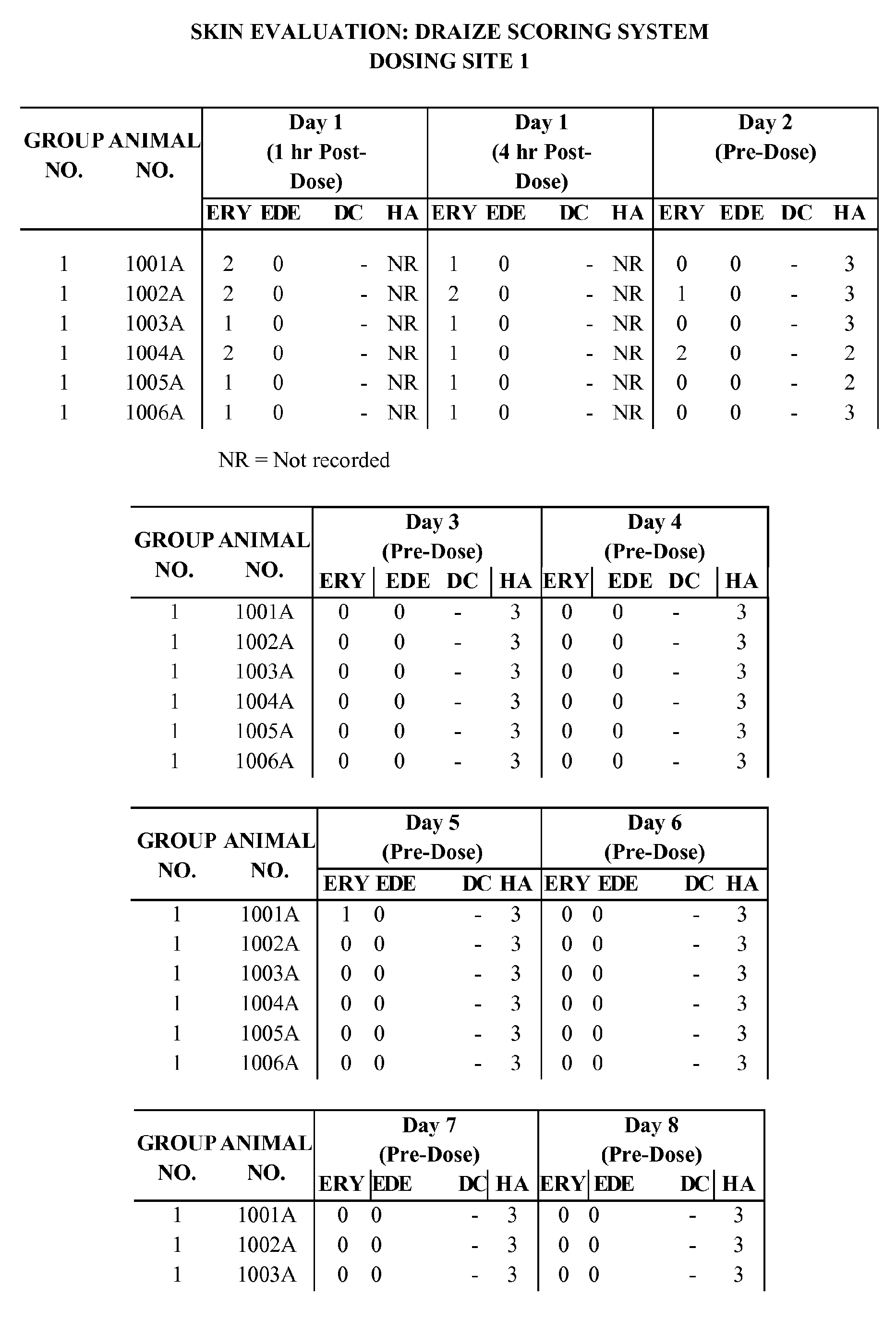

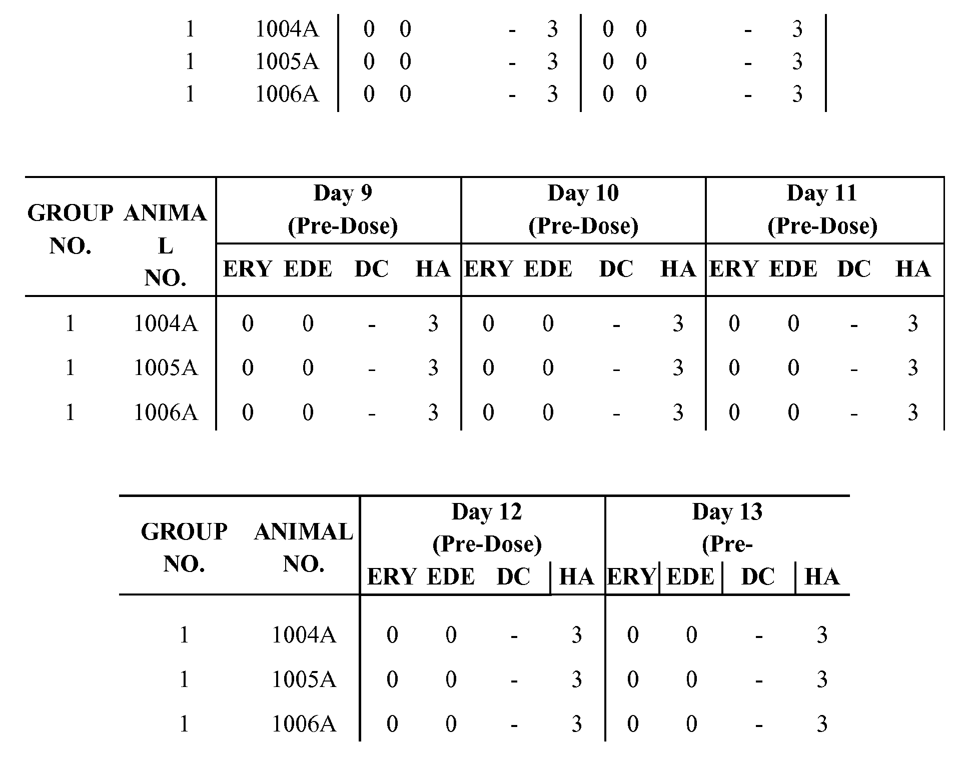

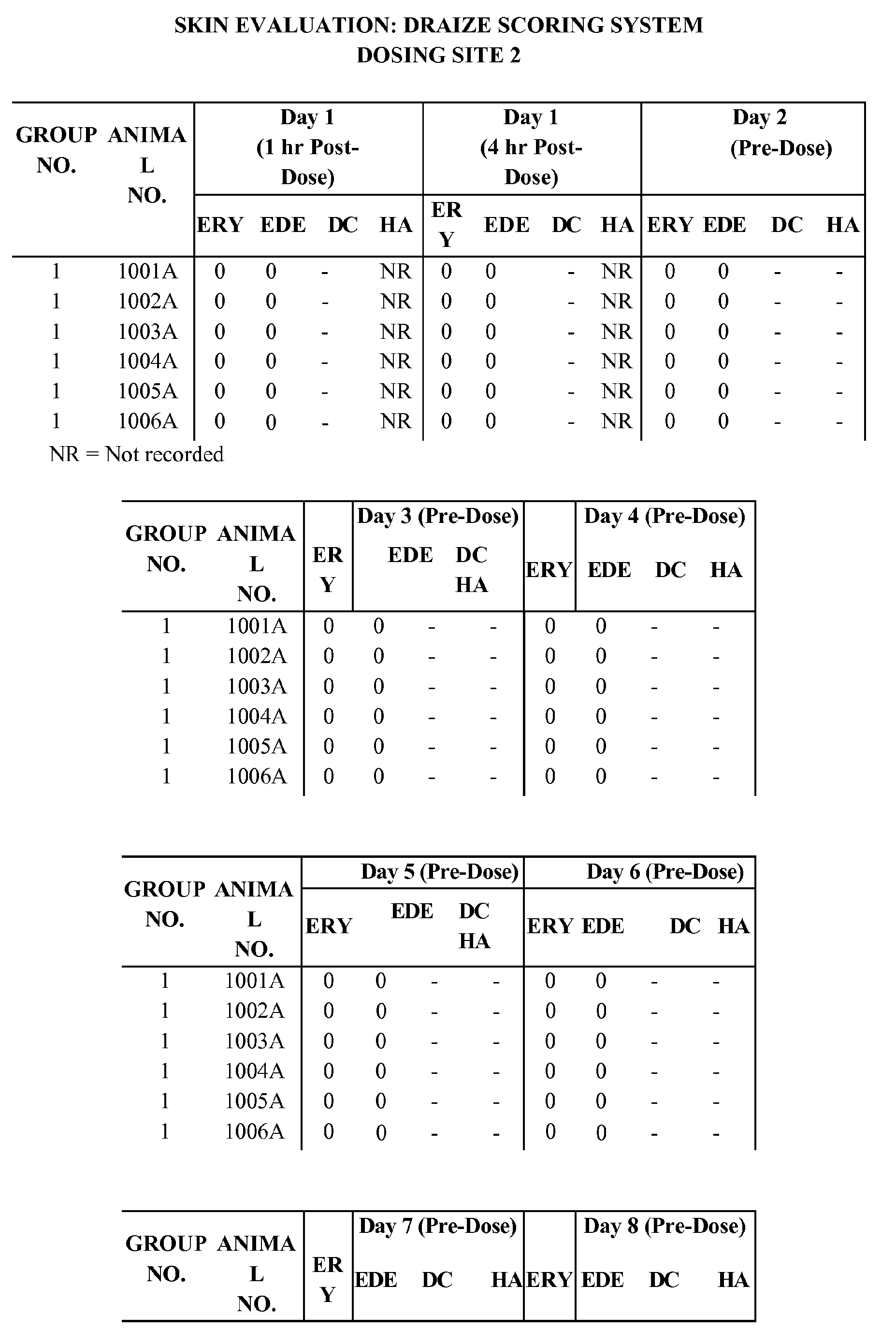

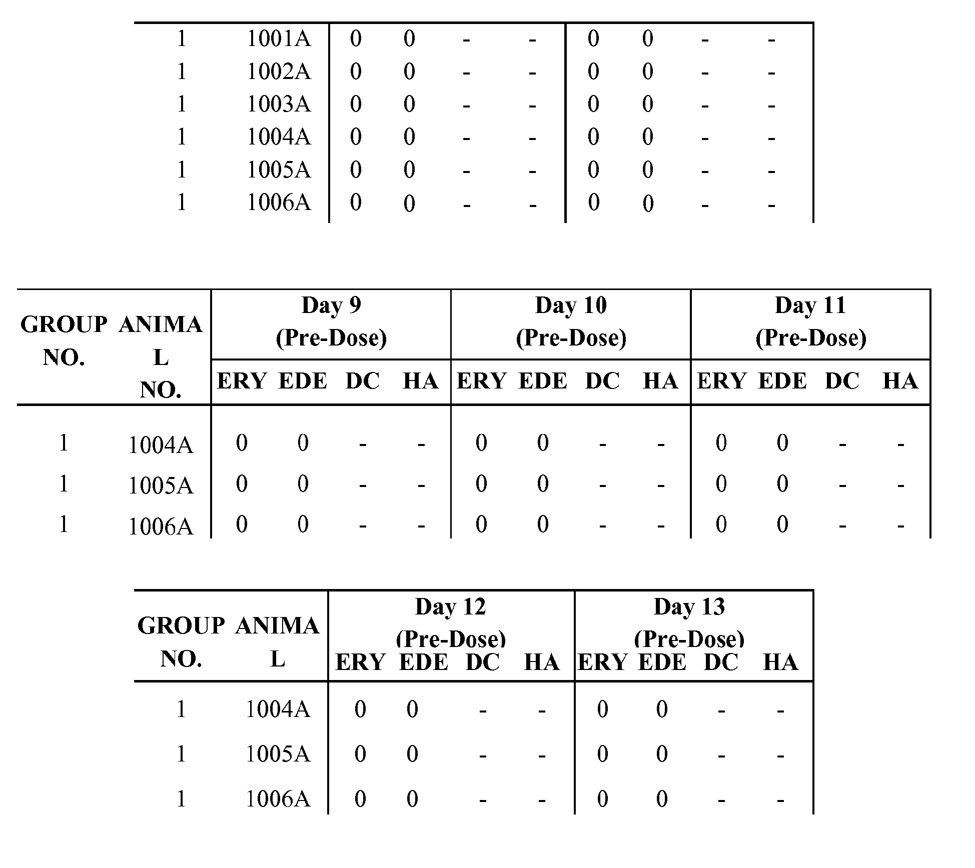

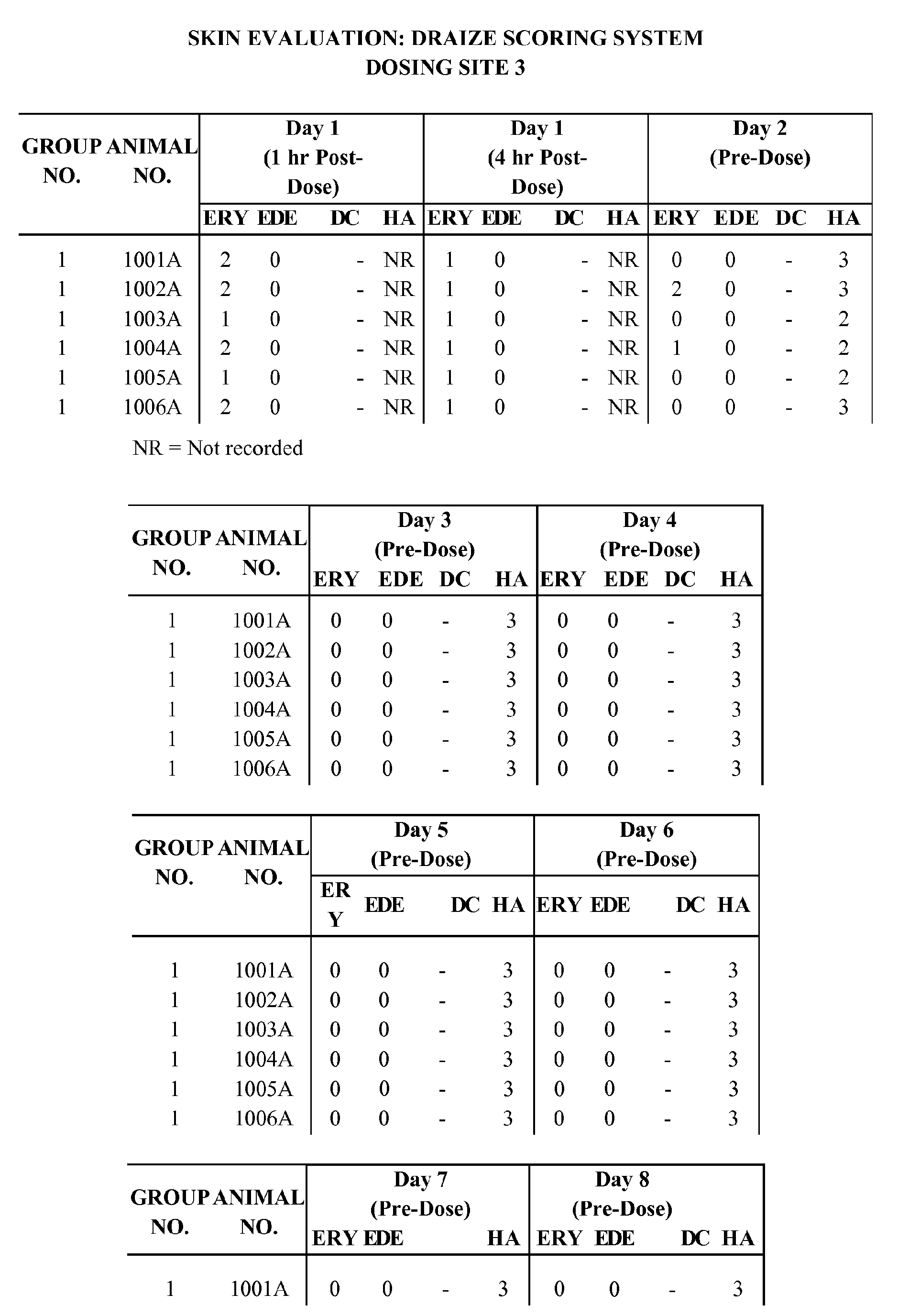

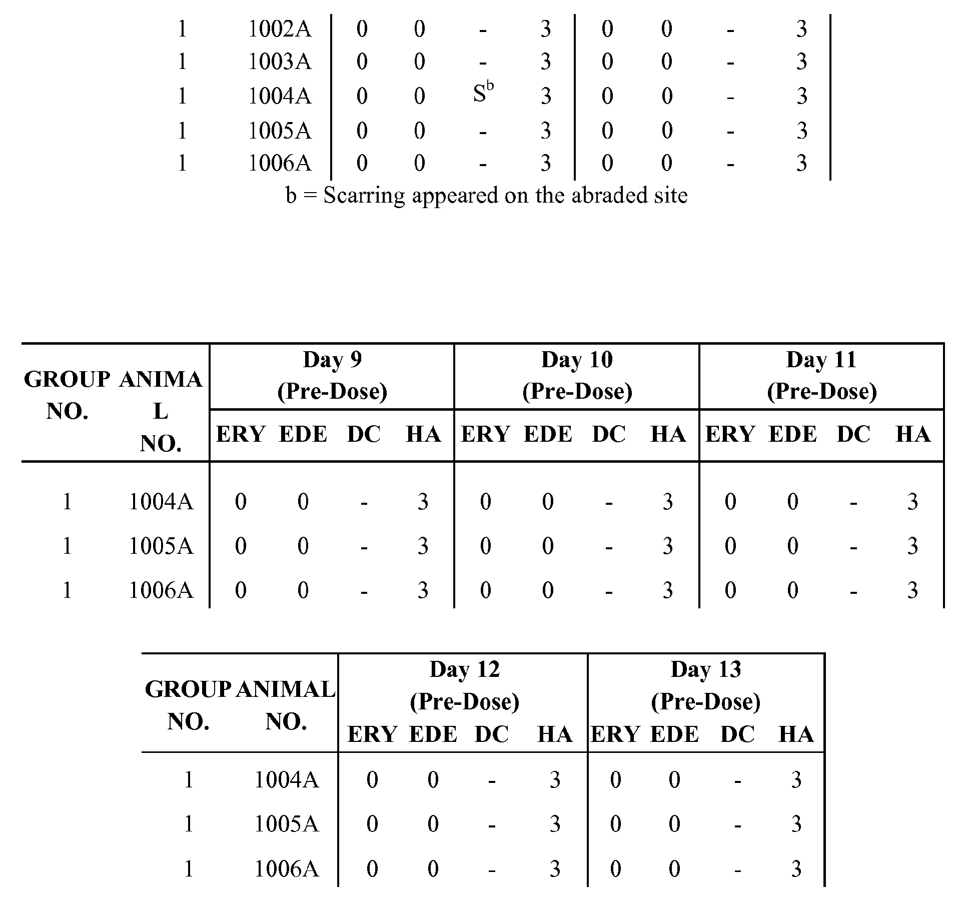

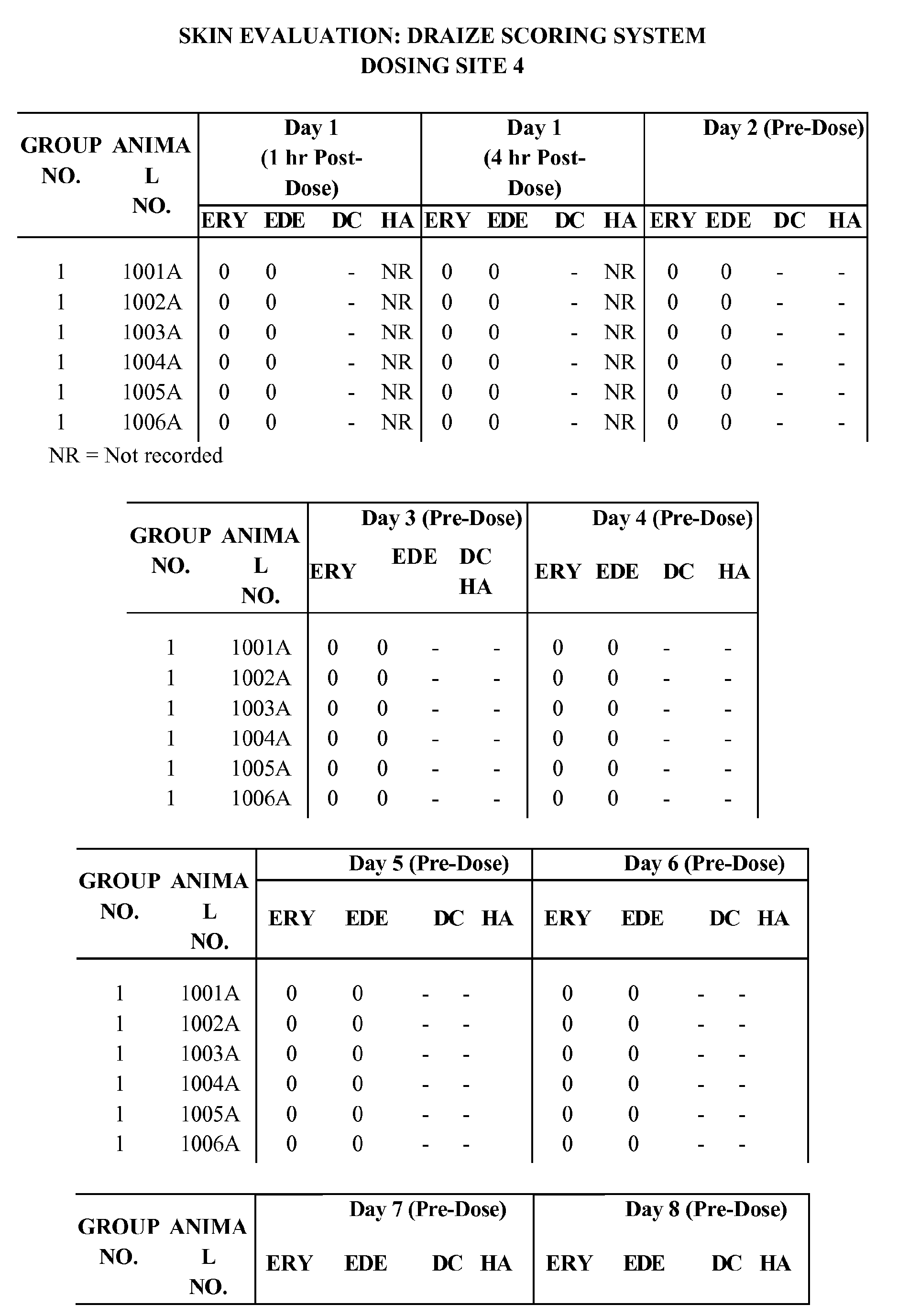

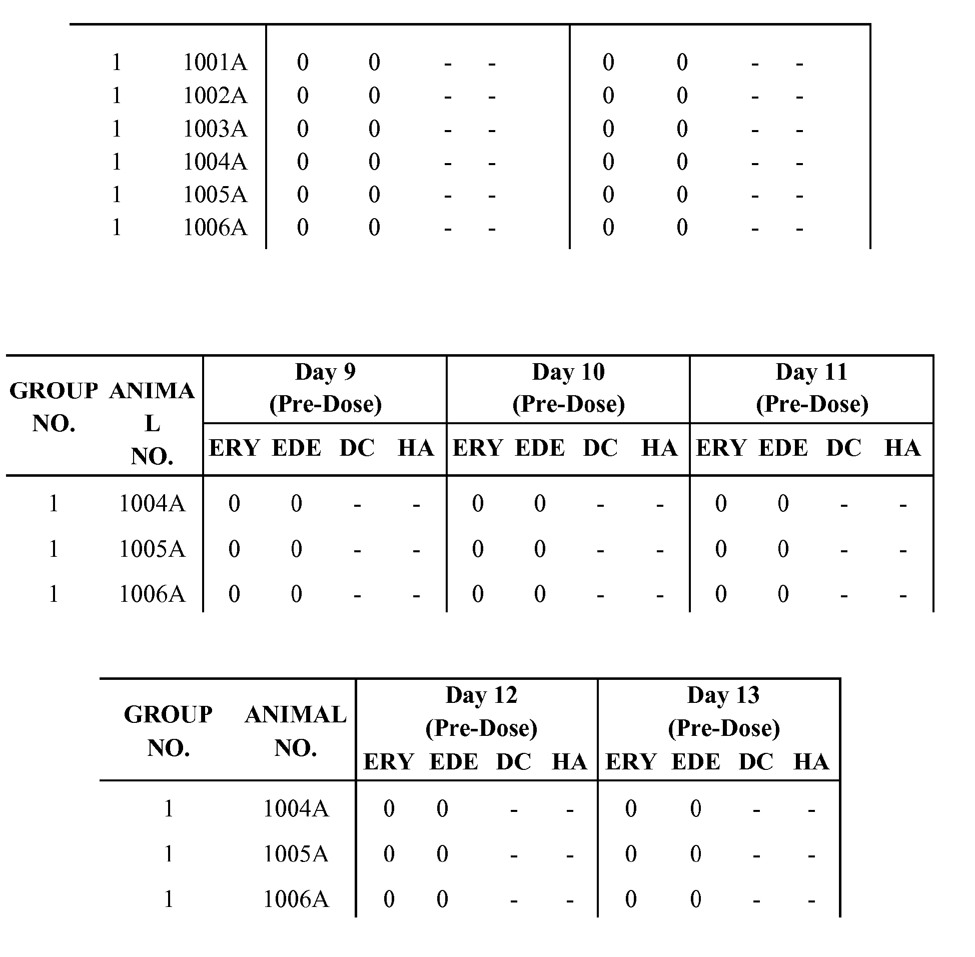

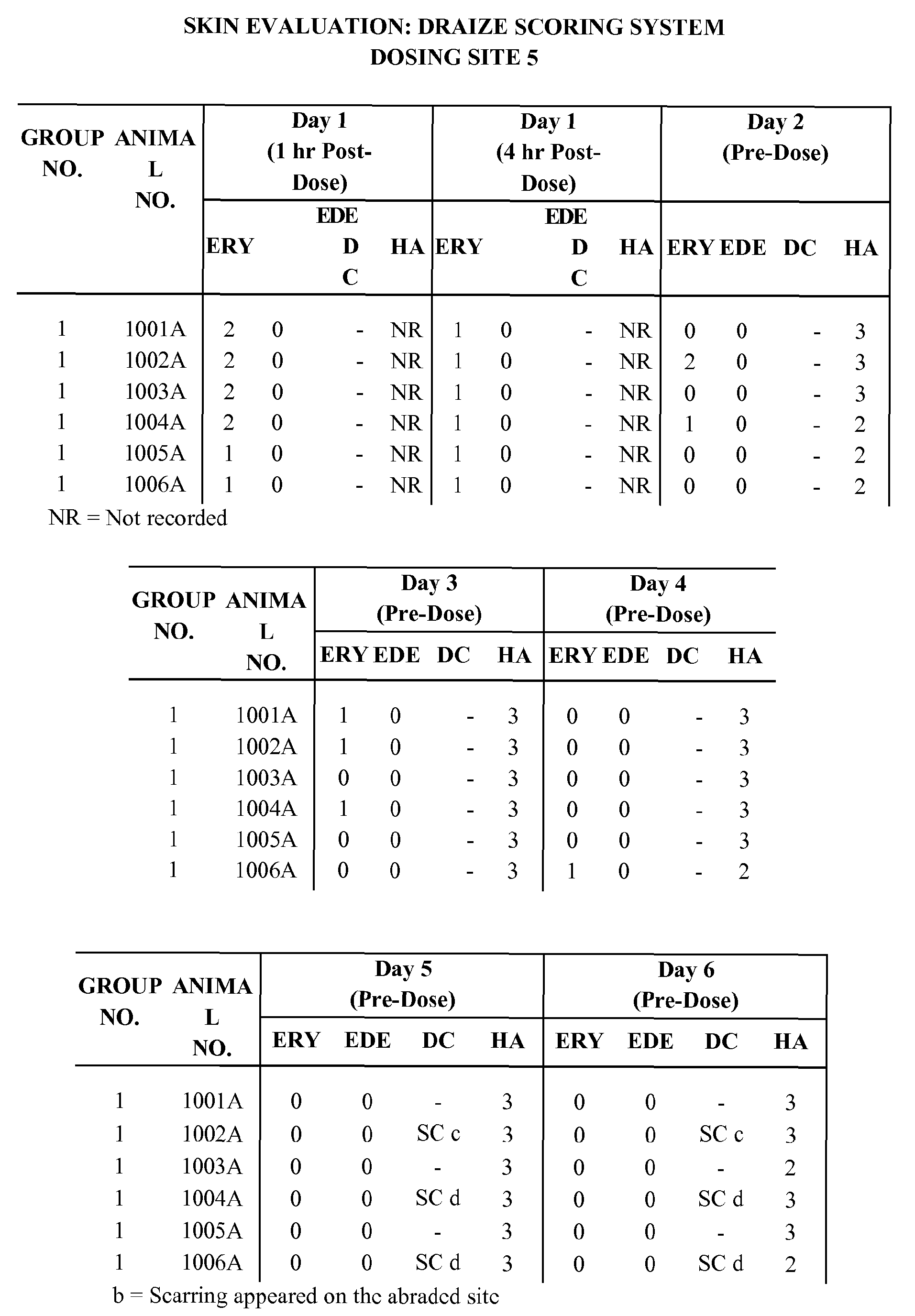

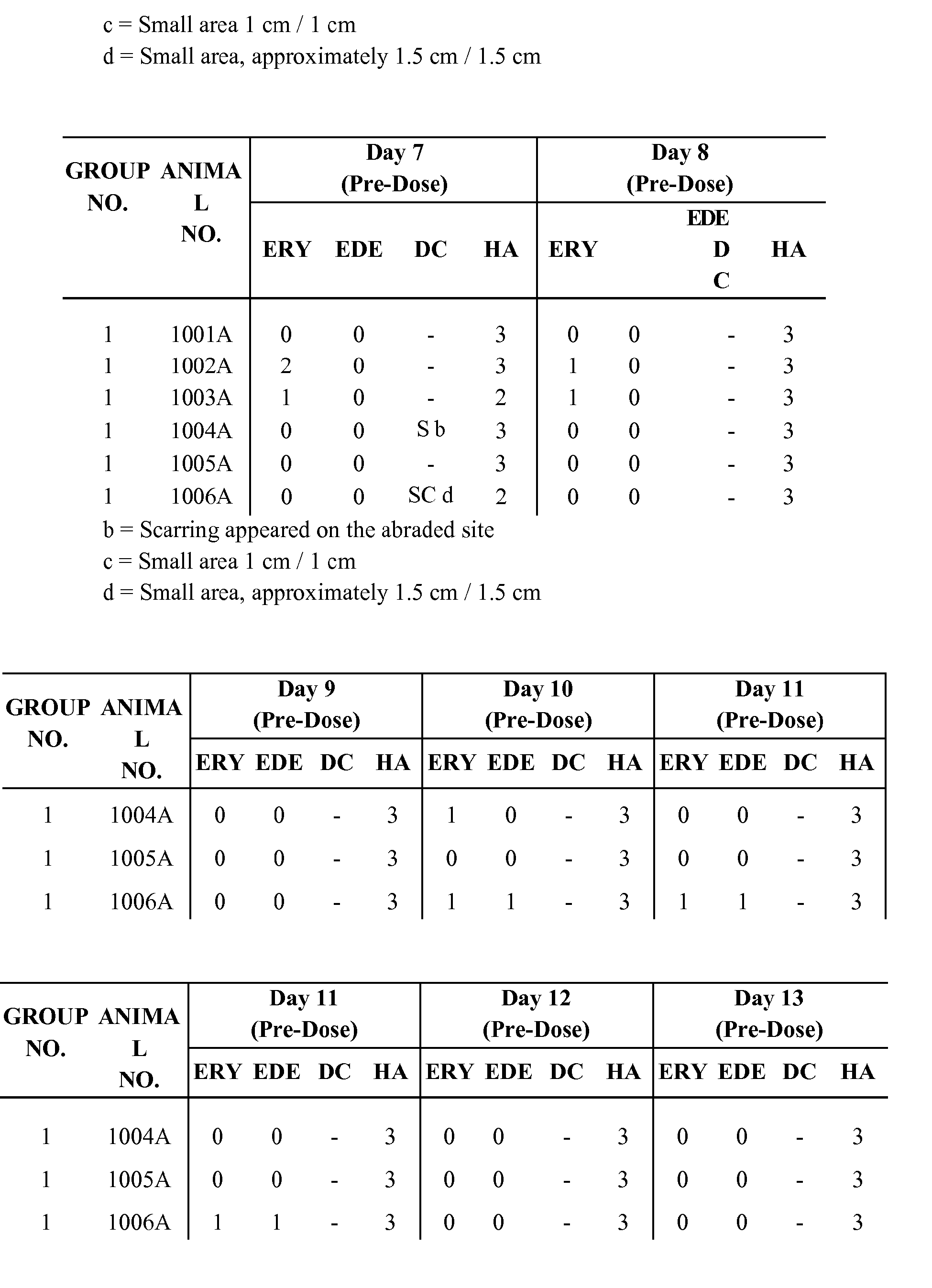

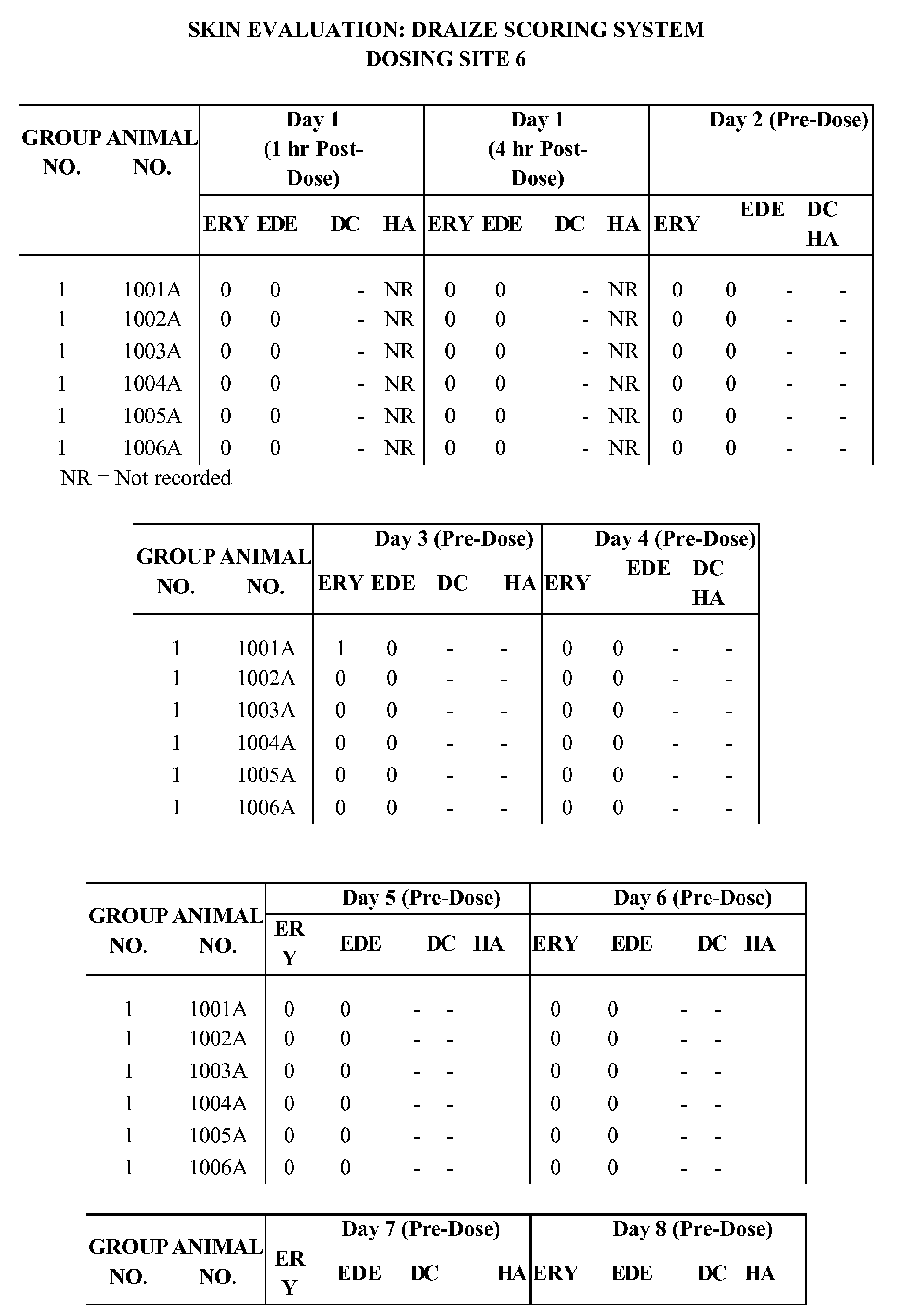

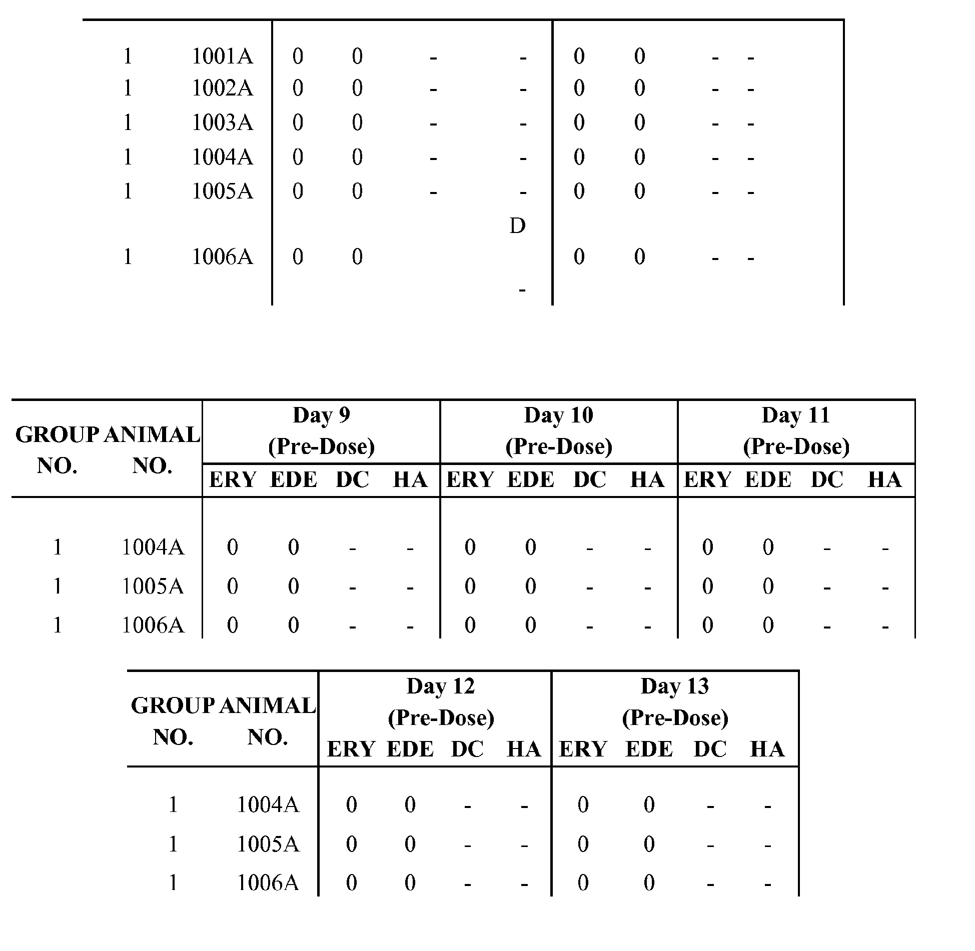

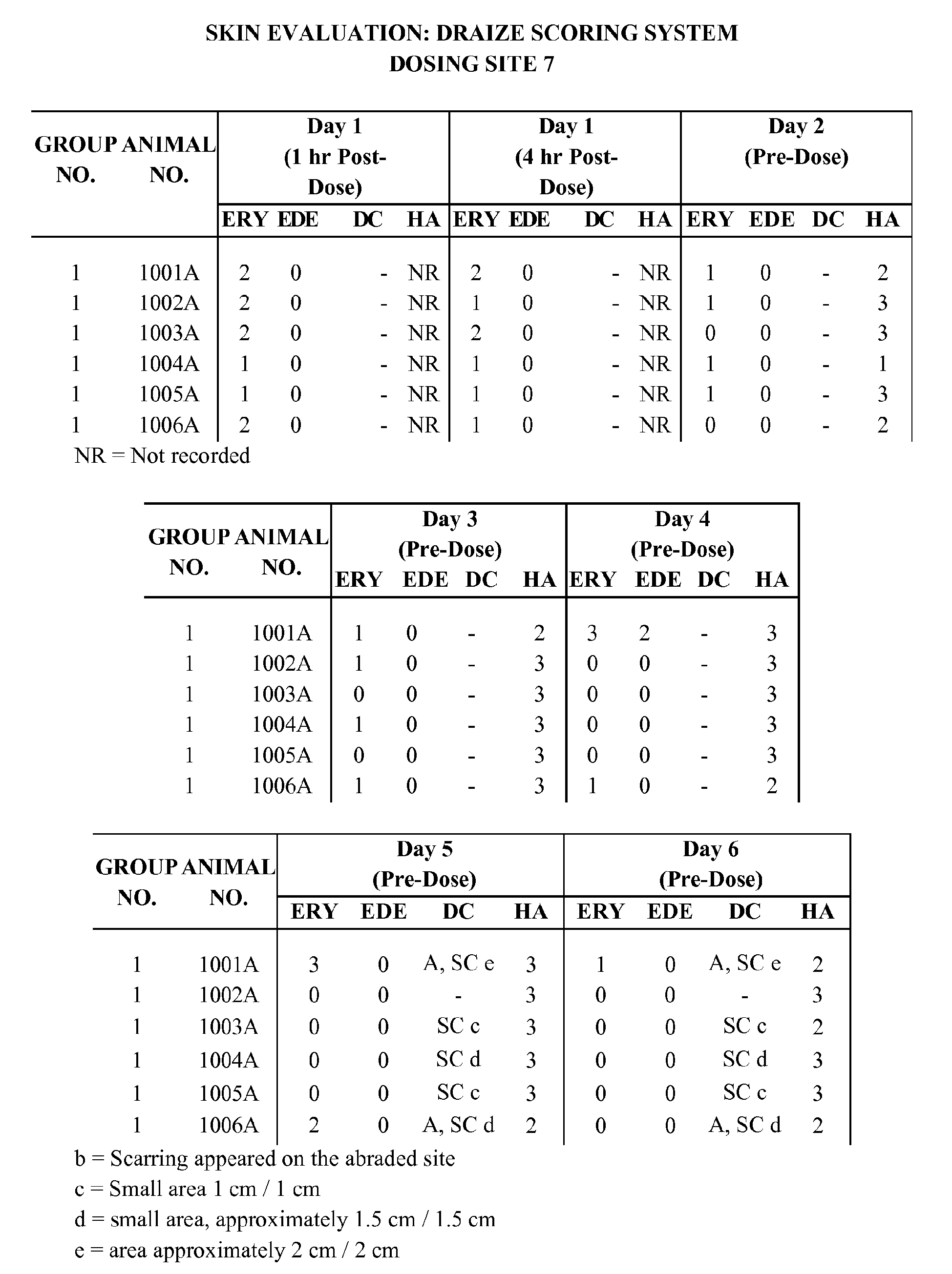

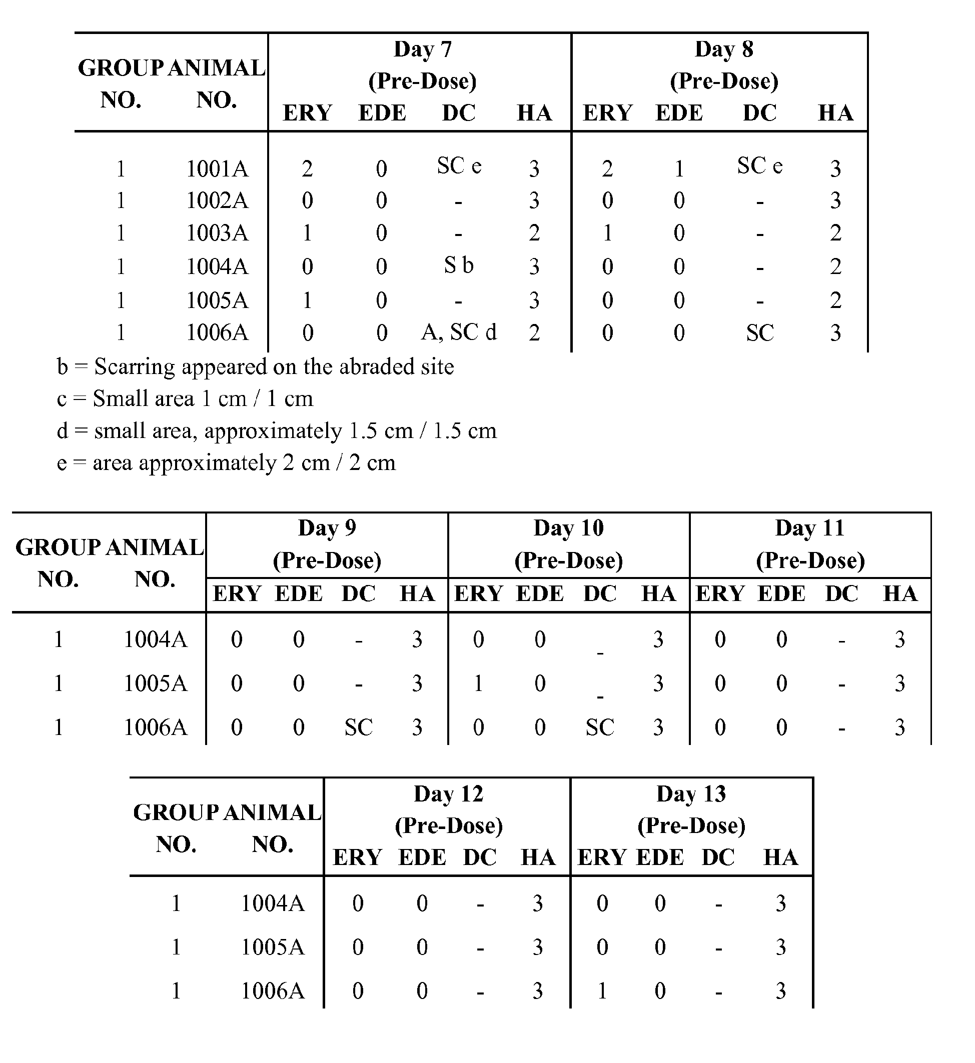

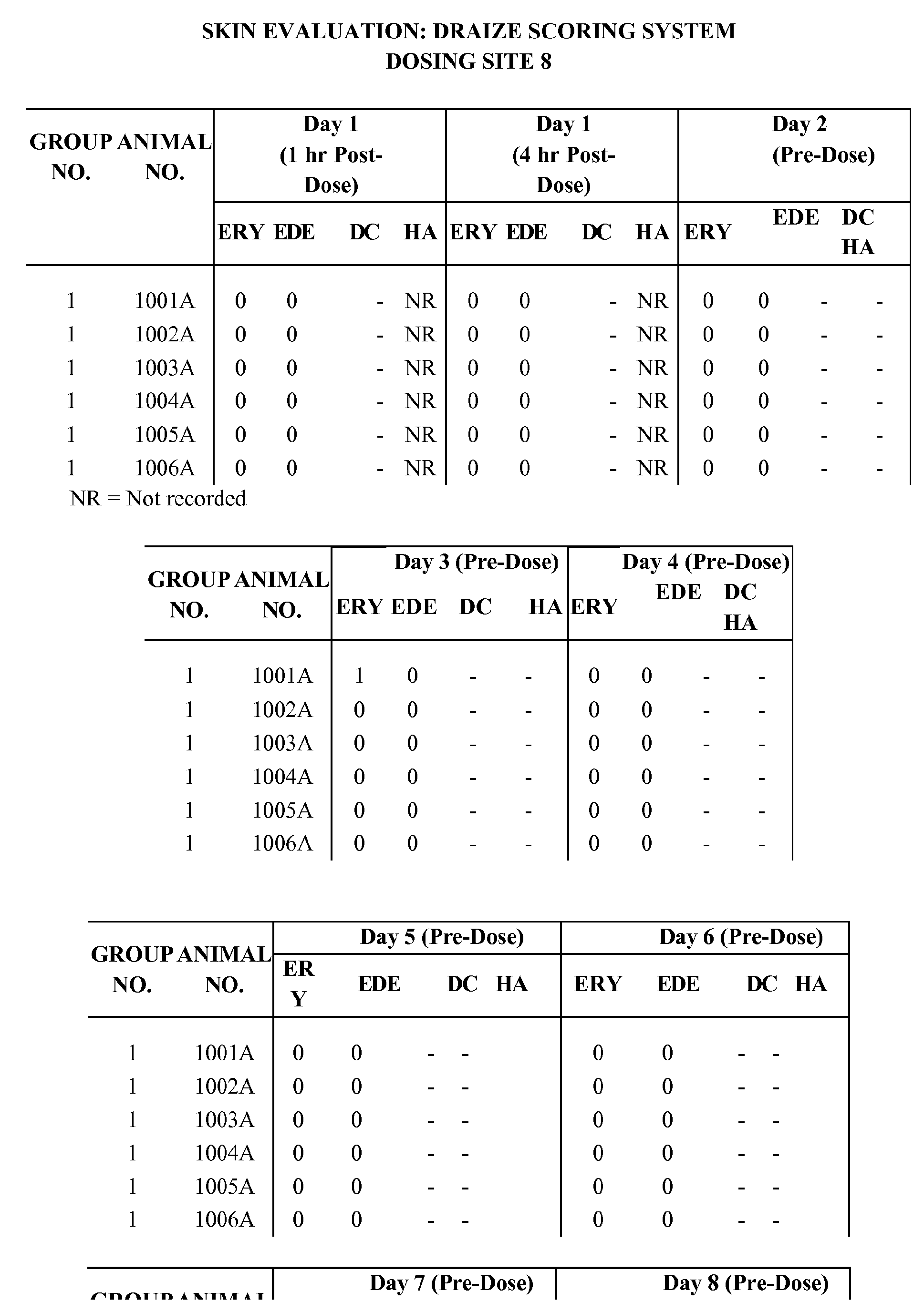

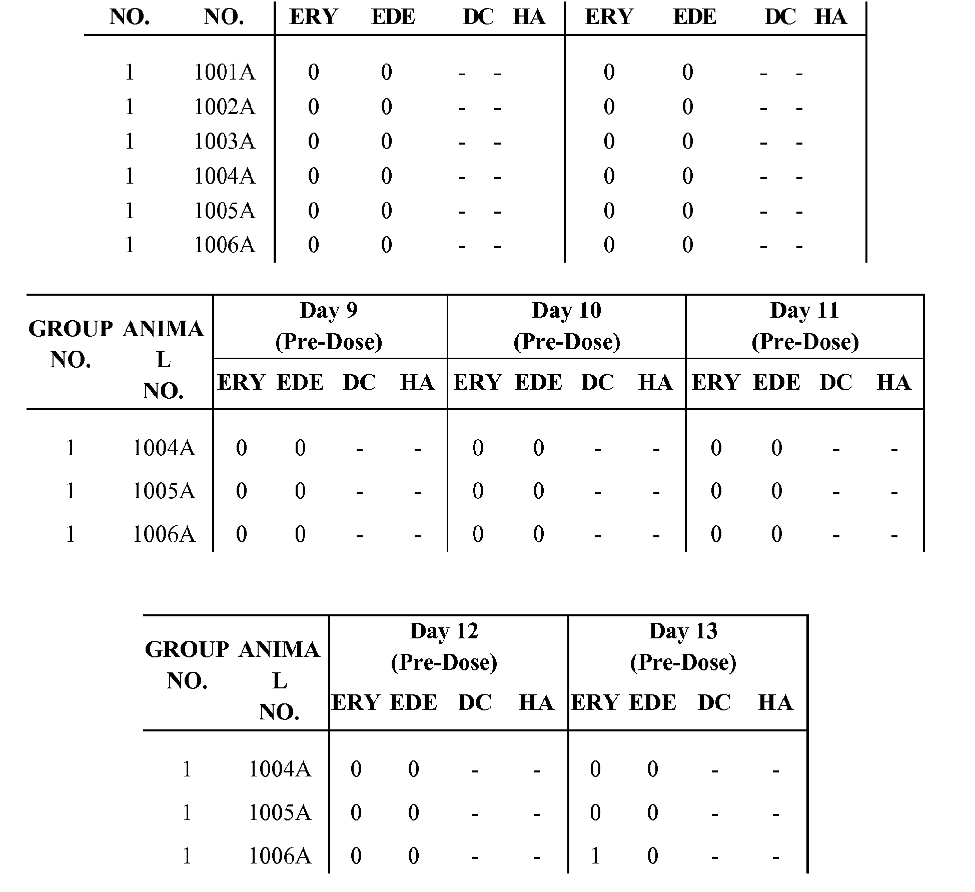

Evaluation of the non abraded placebo control site (site 2) at 1 and 4 hours after the first treatment (day 1) and throughout the observation period did not reveal any dermal changes.

At the abraded site (site 1) treated with placebo, very slight to well-defined erythema was noted at 1 and 4 hours after the first treatment (day 1) but this was related to the abrasion. This dermal change reduced greatly by day 2 and the healing assessment suggested that the majority of animals showed full healing.

With the exception of a few isolated changes, the CM-wh001-treated non abraded sites (sites 4, 6, and 8) showed no reaction to treatment following visual inspection throughout the treatment and observation periods.

Evaluation of the abraded CM-wh001-treated sites on the left flank (sites 3, 5 and 7) showed very slight to well defined erythema at the 1 and 4 hour post dose time points on day 1 as a result of the abrasion that was similar to that seen at the corresponding placebo site.

For abraded sites (site 3) treated with Low dose CM-wh001, these changes subsided through day 2 including the healing assessment and were generally normal for the remainder of the treatment and observation periods. One animal (1004A) had apparent scarring at the site on day 7 only, but this is unlikely to be of significance as it was not noted before or after this occasion.

At the abraded sites (site 5) treated with Mid dose CM-wh001, sites had returned to normal by day 3 with just 3/6 rabbits showing very slight erythema and all animals appearing normal by the healing assessment. However, by day 5 some scabbing was noted in 3/6 animals (between 1 and 1.5 cm square) with one animal showing scaring where the abrasion was made. These changes persisted until day 7, but reduced and, by day 8 (3 days after cessation of dosing), the animals appeared to be back to normal with only 2/6 animals showing very slight erythema. For the remaining animals observed to day 13, there were no more than occasional instances of erythema and edema that were considered to be incidental.

At the abraded sites (site 7) treated with High dose CM-wh001, sites showed progress towards normality with a reduction in the incidence of very slight erythema and the majority of animals appearing normal by the healing assessment. However, by day 5 some scabbing was noted in 5/6 animals (between 1 and 2 cm square) with two animals showing atonia (skin folds defined as a loss of elasticity). By day 7 the number of affected animals had reduced to 3/6 with two showing scabbing and one scarring. Scabbing was seen in these two animals on day 8 and the surviving animal was noted as normal on day 11.

### Macroscopic Findings (See Tables Below)

### Day 8 Terminal Animals

The first 3 rabbits (1001A, 1002A and 1003A) were euthanized on day 8 of the study. Dark discoloration of the abraded skin of the left high dosing site 7 (3/3) and the unabraded skin of the right high dosing site 8 (1/3), exclusively, was noted in the animals.

This gross pathology finding (dark discoloration of the abraded skin) correlated with the microscopic findings of epidermal hyperplasia and/or superficial dermal inflammation. A few dark red areas, considered incidental, were present at one right control dosing site. Dark discoloration of the skin was not present in the abraded skin of the left lower (low dose and mid dose) dosing sites and in the left control site. There were no other gross pathology findings in the abraded or unabraded treated or control sites.

### Day 13 Terminal Animals

Gross pathology findings were not present in the abraded or unabraded treated or control dermal sites.

### Microscopic Findings (See Tables Below)

### Day 8 Terminal Animals

### Abraded Skin (Left Dosing Sites)

At the end of the 2-day observation period following treatment with the test and placebo articles (*i.e.*, day 8), control abraded skin (left dosing site) appeared histologically normal (3/3) and similar to control unabraded skin (right dosing site). This observation suggested healing of the control skin abrasion, with restitution (no hyperplasia) of the epidermis and no evidence of superficial dermal inflammation.

CM-wh001-related, dose-dependent epidermal hyperplasia of an unbreached (reepithelialized) skin, with superficial acute to subacute dermal inflammation, was noted at the abraded dermal sites, as a minimal finding for the low-dose topical application of CM-wh001 (3/3), a minimal to mild finding for the mid dose topical application of CM-wh001 (3/3), and a mild to moderate finding for the high dose topical application of CM-wh001 (3/3). This finding (epidermal hyperplasia, with superficial dermal inflammation) at the left abraded dermal sites did not indicate local toxicity, but rather suggested local tissue irritation or stimulation of epidermal keratinocyte proliferation by the CM-wh001 topical application, possibly related to restored epidermal integrity relative to the normal restorative process.

Since the epidermis appeared unbreached (reepithelialized) by day 8 of the study, this finding (epidermal hyperplasia, with superficial dermal inflammation) also indicated that, with topical application of CM-wh001, the integrity of the abraded skin was restored, albeit by a hyperplastic (and not a restituted epidermis, as the control abraded skin sites). The CM-wh001-related epidermal hyperplasia was further characterized by hyperplasia of basal keratinocytes (basal hyperplasia), often with increased mitotic activity, expansion of the stratum spinosum (acanthosis) and expansion of stratum granulosum (hypergranulosis). Thus, the CM-wh001-related epidermal hyperplasia may also be considered an exaggerated, but favorable, epidermal physiological response, engendered by the topical application of CM-wh001 and which may serve to shore up the strength of the restored epidermis at the site of the healing skin abrasion.

### Unabraded Skin (Right Dosing Sites)

A CM-wh001-related, minimal to mild epidermal hyperplasia of an unbreached (reepithelialized) skin, with or without minimal superficial acute to subacute dermal inflammation, was noted at the unabraded dermal site for the high dose topical application of CM-wh001 (2/3). This finding (epidermal hyperplasia, with superficial dermal inflammation) at the unabraded dermal sites did not indicate local toxicity, but rather suggested local tissue irritation or stimulation of epidermal keratinocyte proliferation by the CM-wh001 topical application.

In comparison with the abraded skin that was treated with high dose topical application of CM-wh001, this finding (epidermal hyperplasia, with superficial dermal inflammation) in the analogous high dose unabraded dermal site was mitigated in severity (minimal to mild for unabraded skin versus mild to moderate for abraded) and in incidence (2/3 for unabraded skin versus 3/3 for abraded skin). This observation suggested that, at high dose topical application of CM-wh001, epidermal hyperplasia at the abraded skin site may have been induced by the concerted (synergistic) influence of skin abrasion ***per se*** and treatment of the abraded skin with CM-wh001.

By day 8 of the study, unabraded dermal sites for the control, low and mid-dose CM-wh001 treatments appeared histologically normal.

### Day 13 Terminal Animals

### Abraded Skin (Left Dosing Sites)

The dose-dependent epidermal hyperplasia of an unbreached (reepithelialized) skin, with superficial acute to subacute dermal inflammation, noted at the CM-wh001-treated abraded dermal sites, had resolved by day 13. These dermal sites appeared histologically normal and similar to the control abraded sites, at the end of the 7-day observation period. This observation suggested healing of the skin abrasion, with restitution of the epidermis and no evidence of superficial dermal inflammation, in the animals retained for the 7-day observation period.

### Unabraded Skin (Right Dosing Sites)

CM-wh001-related, epidermal hyperplasia of an unbreached (reepithelialized) skin, with or without superficial acute to subacute dermal inflammation, noted at the unabraded dermal site for the high dose 8% active topical application of CM-wh001, had resolved by day 13. These resolved dermal sites appeared histologically normal and similar to the control unabraded sites, following the 7-day observation period.

### Additional findings:

A spectrum of changes was observed at the dermal test sites, ranging from no change to resolution of symptoms. This allowed a comparison to be made between the sites at the end of dosing (*i.e.,* day 5) and again after a visual assessment of a return to normal.

Placebo control abraded and non abraded sites showed no dermal changes and normal healing of abrasions. Similarly the unabraded dermal sites at all concentrations (sites 4, 6 and 8) remained generally normal throughout the study.

Evaluation of changes at the dermal test sites of the animals treated daily for 5 days revealed generally no dermal changes at the non abraded sites.

Evaluation of changes at the dermal test sites of the animals treated daily for 5 days revealed very-slight to well-defined erythema at all the abraded dermal test sites (sites 3, 5 and 7). Similar erythema results were observed in test and control sites at the 1 and 4 hour post dose time points on Day 1, but this was a result of the abrasion.

### Treatment with Low dose CM-wh001

For abraded sites treated with low dose CM-wh001 (site 3), the erythema subsided through day 2 as indicated by the healing assessment. The sites were generally normal for the remainder of the treatment and observation periods.

One animal (1004A) had apparent scarring at the site on day 7, but this is unlikely to be of significance as it was not noted before or after this occasion.

### Treatment with Mid dose CM-wh001

At the abraded sites treated with mid dose CM-wh001 (site 5), the skin had returned to normal by day 3 with just 3/6 rabbits showing very slight erythema and all animals appearing normal by the healing assessment.

On day 5, some scabbing (between 1 and 1.5 cm square), was noted in 3/6 animals with one animal showing scaring where the abrasion had been made. These changes persisted to day 7, but then were reduced and, by day 8 (3 days after cessation of dosing), the skin appeared to be back to normal with only 2/6 animals showing very slight erythema. For the remaining animals observed to day 13, there were no more than occasional instances of erythema and edema that were considered to be incidental.

### Treatment with High dose CM-wh001

At the abraded sites treated with high dose CM-wh001 (site 7), skin showed progress towards normality with a reduction in the incidence of erythema and the majority of animals appearing normal by the healing assessment.

By day 5, some scabbing (between 1 and 2 cm square) was noted in 5/6 animals with two animals showing atonia (skin folds defined as a loss of elasticity). By day 7, the number of affected animals had reduced to 3/6 with two showing scabbing and one scarring. Scabbing was seen in two animals on day 8 and the surviving animal was noted as normal on day 11.

### Other

Changes at the abraded dermal test sites necessitated a change to the schedule for termination. Animals and skin histology were assessed on two days: the first on day 8 with a selection of animals representative of the worst and best cases, and the second kill on day 13 with similarly affected animals.

### (i) Day 8

The first 3 rabbits (1001A, 1002A and 1003A) were euthanized on day 8 of the study. Dark discoloration of the abraded skin of the left high dosing site 7 (3/3) and the unabraded skin of the right high dosing site 8 (1/3), exclusively, was noted, macroscopically, in the animals.

This gross pathology finding (dark discoloration of the abraded skin) correlated with the microscopic findings of epidermal hyperplasia and/or superficial dermal inflammation. A few dark red areas, considered incidental, were present at one right control dosing site (site 2). Dark discoloration of the skin was not present in the abraded skin of the left low dose (site 3) and mid dose (site 3) dosing sites and in the left control site (site 1). There were no other gross pathology findings in the abraded or unabraded treated or control dermal sites. Similarly, gross pathology findings were not present in the abraded or unabraded treated or control dermal sites of the animals assessed on day 13.

By day 8 of the study, control abraded skin (site 1) appeared histologically normal in 3/3 animals and similar to control unabraded skin (site 2). This observation suggested healing of the control skin abrasion, with restitution (no hyperplasia) of the epidermis and no evidence of superficial dermal inflammation.

CM-wh001-related, dose-dependent epidermal hyperplasia of an unbreached (reepithelialized) skin, with superficial acute to subacute dermal inflammation, was noted at the abraded dermal sites as follows:
- a minimal finding for the low-dose topical application of CM-wh001 (3/3),
- a minimal to mild finding for the mid dose topical application of CM-wh001 (3/3), and
- a mild to moderate finding for the high dose topical application of CM-wh001 (3/3).

This finding (epidermal hyperplasia, with superficial dermal inflammation) at the left abraded dermal sites did not indicate local toxicity, but rather suggested local tissue irritation or stimulation of epidermal keratinocyte proliferation by the CM-wh001 topical application. Since the epidermis appeared unbreached (reepithelialized) by day 8 of the study, this finding (epidermal hyperplasia, with superficial dermal inflammation) also indicated that, with topical application of CM-wh001, the integrity of the abraded skin was restored, albeit hyperplastic (and not a restituted epidermis, as the control abraded skin sites). Hyperplastic restoration, in opposition to restituted epidermis relative to a wound, speaks to a phenomenon of further strengthening of the epidermis at the wound site, beyond the normal restorative process.

The CM-wh001-related epidermal hyperplasia was further characterized by hyperplasia of basal keratinocytes (basal hyperplasia), often with increased mitotic activity, expansion of the stratum spinosum (acanthosis) and expansion of stratum granulosum (hypergranulosis). Thus, the CM-wh001-related epidermal hyperplasia may be considered an exaggerated, but favorable, epidermal physiological response, engendered by the topical application of CM-wh001 which may serve to shore up the strength of the restored epidermis at the site of the healing skin abrasion.

Unabraded skin (right dosing sites) treated with the high dose: CM-wh001-related, minimal to mild epidermal hyperplasia of skin, with or without minimal superficial acute to subacute dermal inflammation, was observed in 2 out of 3 animals. This finding did not indicate local toxicity, but rather, suggested local tissue irritation or stimulation of epidermal keratinocyte proliferation by the CM-wh001 topical application.

In comparison with the abraded skin that was treated with high dose topical application of CM-wh001, this finding (epidermal hyperplasia, with superficial dermal inflammation) in the analogous high dose unabraded dermal site was mitigated in severity (minimal to mild for unabraded skin versus mild to moderate for abraded) and in incidence (2/3 for unabraded skin versus 3/3 for abraded skin).

This observation suggested that high dose topical application of CM-wh001 induced epidermal hyperplasia at the abraded skin site indicating enhanced restoration of the epidermis beyond that of the normal restorative process. Enhanced epidermal restoration results in an epidermal layer with greater resistance to further insult.

On day 8, unabraded dermal sites for the control, low and mid dose CM-wh001 treatments appeared histologically normal.

Histopathology revealed that at the end of the observation period (*i.e.,* day 8), there was no evidence of local tissue (skin) toxicity in any of the animals. Overall, the results on day 8 suggested CM-wh001-induced local tissue irritation or stimulation of epidermal keratinocyte proliferation (hyperplasia) or an exaggerated, but desirable, epidermal physiological response, engendered by the topical application of CM-wh001.

### (ii) Day 13

Animals 1004A, 1005A and 1006A were euthanized on day 13 of the study.

**Abraded sites**: By day 13 of the study, the dose-dependent epidermal hyperplasia (superficial acute to subacute dermal inflammation) of unbreached (reepithelialized) skin at the CM-wh001-treated abraded dermal sites, had resolved.

These dermal sites appeared histologically normal and similar to the control abraded sites. This observation suggested that CM-wh001 induced healing of the skin abrasion, with restitution of the epidermis and no evidence of superficial dermal inflammation.

**Unabraded sites**: For the right dosing sites, CM-wh001-related epidermal hyperplasia of unbreached (reepithelialized) skin (with or without superficial acute to subacute dermal inflammation) observed for the high dose topical application of CM-wh001, had resolved. These resolved dermal sites appeared histologically normal and similar to the control unabraded sites.

Topical application of CM-wh001 did not result in mortality or test article related changes in the endpoint parameters.

On day 13 of the study, there was resolution of epidermal hyperplasia of unbreached (reepithelialized) skin and superficial acute to subacute dermal inflammation, at the CM-wh001-treated abraded or unabraded dermal sites, which indicated healing of the skin abrasion with restitution of the epidermis and no evidence of superficial dermal inflammation.

Histopathology revealed that at the end of the observation periods (*i.e.,* day 13), there was no evidence of local tissue (skin) toxicity in any of the animals. By day 13, there was resolution of epidermal hyperplasia of unbreached (reepithelialized) skin and superficial acute to subacute dermal inflammation at the CM-wh001-treated abraded or unabraded dermal sites, which indicated healing of the skin abrasion, with restitution of the epidermis and no evidence of superficial dermal inflammation.

### CONCLUSION

A topical application of the test article, CM-wh001 to each side of the rabbit's dorsum (with the left side abraded and the right side unabraded) for 5 days and following which a 2-day observation period was allowed for the first 3 rabbits (1001A, 1002A and 1003A), and a 7-day observation period for the remaining 3 rabbits (1004A, 1005A and 1006A), demonstrated the following:

On days 8 and 13 of the study, there was no evidence of local tissue (skin) toxicity in any of the animals.

Day 8 of the study: control abraded skin (left dosing site) appeared histologically normal (3/3) and similar to control unabraded skin (right dosing site)(3/3); and this observation suggested healing of the control skin abrasion, with restitution (no hyperplasia) of the epidermis and no evidence of superficial dermal inflammation.

Day 8 of the study: a CM-wh001-related, dose-dependent epidermal hyperplasia of an unbreached (reepithelialized) skin, with superficial acute to subacute dermal inflammation at the abraded skin site, was noted as a minimal finding for the low-dose topical application of CM-wh001 (3/3), a minimal to mild finding for the mid dose topical application of CM-wh001 (3/3), and a mild to moderate finding for the high dose topical application of CM-wh001 (3/3), and which suggested CM-wh001- induced local tissue irritation or stimulation of epidermal keratinocyte proliferation (hyperplasia); the CM-wh001-related epidermal hyperplasia may, also, be considered an exaggerated, but favorable, epidermal physiological response, engendered by the topical application of CM-wh001 and which may serve to shore up the strength of the restored epidermis at the site of the healing skin abrasion.

Day 8 of the study: histological evidence suggested that, at high dose topical application of CM-wh001, epidermal hyperplasia at the abraded skin site may have been induced by the concerted (synergistic) influence of skin abrasion per se and treatment of the abraded skin with CM-wh001.

Day 8 of the study: CM-wh001-related, minimal to mild epidermal hyperplasia of an unbreached (reepithelialized) skin, with or without minimal superficial acute to subacute dermal inflammation, was noted at the unabraded skin site for the high dose topical application of CM-wh001 (2/3) only.

Day 13 of the study: there was resolution of epidermal hyperplasia of an unbreached (reepithelialized) skin and superficial acute to subacute dermal inflammation, at the CM-wh001-treated abraded or unbraded skin sites; this resolution indicated healing of the skin abrasion, with restitution of the epidermis and no evidence of superficial dermal inflammation.

A CM-wh001-related, dose-dependent epidermal hyperplasia of an unbreached (reepithelialized) skin, with superficial acute to subacute dermal inflammation at the abraded dermal sites, was noted by day 8 as a minimal finding for the low-dose topical application of CM-wh001 (3/3), a minimal to mild finding for the mid dose topical application of CM-wh001 (3/3), and a mild to moderate finding for the high dose topical application of CM-wh001 (3/3). These results suggested CM-wh001 induced local tissue irritation or stimulation of epidermal keratinocyte proliferation (hyperplasia). The CM-wh001-related epidermal hyperplasia may also be considered an exaggerated, but favorable, epidermal physiological response engendered by the topical application of CM-wh001 and which may serve to shore up the strength of the restored epidermis at the site of the healing skin abrasion, beyond that of the normal restorative process.

Histological evidence by day 8 suggested that, at high dose topical application of CMwh001, epidermal hyperplasia at the abraded dermal site may have been induced by the concerted (synergistic) influence of skin abrasion per se and treatment of the abraded skin with CM-wh001. Furthermore, CM-wh001-related, minimal to mild epidermal hyperplasia of an unbreached (reepithelialized) skin, with or without minimal superficial acute to subacute dermal inflammation, was noted at the unabraded dosing site for the high dose topical application of CM-wh001 (2/3) only.

By day 13 of the study, there was resolution of epidermal hyperplasia of an unbreached (reepithelialized) skin and superficial acute to subacute dermal inflammation, at the CM-wh001-treated abraded or unabraded dermal sites, which indicated healing of the skin abrasion, with restitution of the epidermis and no evidence of superficial dermal inflammation.

Overall, a CM-wh001-related, dose-dependent epidermal hyperplasia of an unbreached (reepithelialized) skin, with superficial acute to subacute dermal inflammation at the abraded dermal sites, noted by day 8, suggested CM-wh001-induced local tissue irritation or stimulation of epidermal keratinocyte proliferation (hyperplasia) or an exaggerated, but desirable, epidermal physiological response, engendered by the topical application of CM-wh001. By day 13, there was resolution of epidermal hyperplasia of an unbreached (reepithelialized) skin and superficial acute to subacute dermal inflammation, at the CM-wh001-treated abraded or unabraded dermal sites, which indicated healing of the skin abrasion, with restitution of the epidermis and no evidence of superficial dermal inflammation.

### Incidence of Gross Pathology - Terminal Day 8

| | |
|---|---|
| Number of Animals on Study | 3 |
| Number of Animals Completed | (3) |

| LEFT CONTROL DOSING SITE (**Site 1**) | |
|---|---|
| Submitted | (3) |
| No Visible Lesions | 3 |

| LEFT LOW DOSING SITE (**Site 3**) | |
|---|---|
| Submitted | (3) |
| No Visible Lesions | 3 |

| LEFT MID DOSING SITE (**Site 5**) | |
|---|---|
| Submitted | (3) |
| No Visible Lesions | 3 |

| LEFT HIGH DOSING SITE (**Site 7**) | |
|---|---|
| Submitted | (3) |
| No Visible Lesions | 0 |
| Dark discoloration; Skin | 3 |

| RIGHT CONTROL DOSING SITE (**Site 2**) | |
|---|---|
| Submitted | (3) |
| No Visible Lesions | 2 |
| Dark area; red; Skin; few | 1 |

| RIGHT LOW DOSING SITE (**Site 4**) | |
|---|---|
| Submitted | (3) |
| No Visible Lesions | 3 |

| RIGHT MID DOSING SITE (**Site 6**) | |
|---|---|
| Submitted | (3) |
| No Visible Lesions | 3 |

| RIGHT HIGH DOSING SITE (**Site 8**) | |
|---|---|
| Submitted | (3) |
| No Visible Lesions | 2 |
| Dark discoloration; Skin | 1 |

### INCIDENCE OF GROSS PATHOLOGY - Terminal Day 13

| | |
|---|---|
| Number of Animals on Study : | 3 |
| Number of Animals Completed: | (3) |

| **LEFT CONTROL DOSING SITE (Site 1)** | |
|---|---|
| Submitted | (3) |
| No Visible Lesions | 3 |

| **LEFT LOW DOSING SITE (Site 3)** | |
|---|---|
| Submitted | (3) |
| No Visible Lesions | 3 |

| **LEFT MID DOSING SITE (Site 5)** | |
|---|---|
| Submitted | (3) |
| No Visible Lesions | 3 |

| **LEFT HIGH DOSING SITE (Site 7)** | |
|---|---|
| Submitted | (3) |
| No Visible Lesions | 3 |

| **RIGHT CONTROL DOSING SITE (Site 2)** | |
|---|---|
| Submitted | (3) |
| No Visible Lesions | 3 |

| **RIGHT LOW DOSING SITE (Site 4)** | |
|---|---|
| Submitted | (3) |
| No Visible Lesions | 3 |

| **RIGHT MID DOSING SITE (Site 6)** | |
|---|---|
| Submitted | (3) |
| No Visible Lesions | 3 |

| **RIGHT HIGH DOSING SITE (Site 8)** | |
|---|---|
| Submitted | (3) |
| No Visible Lesions | 3 |

### INCIDENCE OF HISTOPATHOLOGY - Terminal Day 8

| | |
|---|---|
| Observations: Neo-Plastic and Non Neo-Plastic | |
| Number of Animals on | 3 |
| Study: | (3) |

| **LEFT CONTROL DOSING SITE (site 1)** | |
|---|---|
| Examined | (3) |
| Within Normal Limits | 3 |

| **LEFT LOW DOSING SITE (site 3)** | |
|---|---|
| Examined | (3) |
| Within Normal Limits | 0 |
| Hyperplasia, epidermal | (3) |
| minimal | 3 |
| Acanthosis | (3) |
| minimal | 3 |
| Dermal inflammation, superficial | (3) |
| minimal | 2 |
| mild | 1 |
| Hyperplasia, basal | (3) |
| minimal | 3 |
| Hypergranulosis | (3) |
| minimal | 3 |

| **LEFT MID DOSING SITE (site** 5) | |
|---|---|
| Examined | (3) |
| Within Normal Limits | 0 |
| Hyperplasia, epidermal | (3) |
| minimal | 1 |
| mild | 2 |
| Dermal inflammation, superficial | (3) |
| minimal | 1 |
| mild | 2 |
| Acanthosis | (3) |
| minimal | 1 |
| mild | 2 |
| Hypergranulosis | (3) |
| minimal | 1 |
| mild | 2 |
| Hyperplasia, basal | (3) |
| minimal | 1 |
| mild | 2 |

| **LEFT HIGH DOSING SITE (site 7)** | |
|---|---|
| Examined | (3) |
| Within Normal Limits | 0 |
| Hyperplasia, epidermal | (3) |
| mild | 2 |
| moderate | 1 |
| Dermal inflammation, superficial | (3) |
| minimal | 1 |
| mild | 2 |
| Acanthosis | (3) |
| mild | 2 |
| moderate | 1 |
| Hyperplasia, basal | (3) |
| mild | 2 |
| moderate | 1 |
| Hypergranulosis | (3) |
| mild | 2 |
| moderate | 1 |

| **RIGHT CONTROL DOSING SITE (site 2)** | |
|---|---|
| Examined | (3) |
| Within Normal Limits | 3 |

| **RIGHT LOW DOSING SITE (site 4)** | |
|---|---|
| Examined | (3) |
| Within Normal Limits | 3 |

| **RIGHT MID DOSING SITE (site 6)** | |
|---|---|
| Examined | (3) |
| Within Normal Limits | 3 |

| **RIGHT HIGH DOSING SITE (site 8)** | |
|---|---|
| Examined | (3) |
| Within Normal Limits | 1 |
| Hyperplasia, epidermal | (2) |
| minimal | 1 |
| mild | 1 |
| Acanthosis | (2) |
| minimal | 1 |
| mild | 1 |
| Hyperplasia, basal | (2) |
| minimal | 1 |
| mild | 1 |
| Hypergranulosis | (2) |
| minimal | 1 |
| mild | 1 |
| Dermal inflammation, superficial | (1) |
| minimal | 1 |

### INCIDENCE OF HISTOPATHOLOGY - Terminal Day 13

| | |
|---|---|
| Observations: Neo-Plastic and Non Neo-Plastic | |
| Number of Animals on Study : | 3 |
| Number of Animals Completed: | (3) |

| **LEFT CONTROL DOSING SITE (site 1)** | |
|---|---|
| Examined | (3) |
| Within Normal Limits | 3 |

| **LEFT LOW DOSING SITE (site 3)** | |
|---|---|
| Examined | (3) |
| Within Normal Limits | 3 |

| **LEFT MID DOSING SITE (site 5)** | |
|---|---|
| Examined | (3) |
| Within Normal Limits | 3 |

| **LEFT HIGH DOSING SITE (site 7)** | |
|---|---|
| Examined | (3) |
| Within Normal Limits | 3 |

| **RIGHT CONTROL DOSING SITE (site 2)** | |
|---|---|
| Examined | (3) |
| Within Normal Limits | 3 |

| **RIGHT LOW DOSING SITE (site 4)** | |
|---|---|
| Examined | (3) |
| Within Normal Limits | 3 |

| **RIGHT MID DOSING SITE (site 6)** | |
|---|---|
| Examined | (3) |
| Within Normal Limits | 3 |

| **RIGHT HIGH DOSING SITE (site 8)** | |
|---|---|
| Examined | (3) |
| Within Normal Limits | 3 |

### INDIVIDUAL ANIMAL DATA (Animal Ref.: 1001A)

Group: 1
Sex: Male
Species: Rabbit
Strain: New Zealand White
Test Material: Dose: Group 1 Route: Dermal Study Type: Tolerance Study Date of Death: 12/01/11 Study Day No. (Week): 8 (2) Mode of Death: Terminal Date of Necropsy: 12/01/11 ** NECROPSY COMPLETE **
** EXAMINATION COMPLETE **
Terminal Body Weight: 3 kg

| **Gross Pathology Observations** | **Correlated with:** |
|---|---|
| LEFT HIGH DOSING SITE | |
| Skin: Dark discoloration (TGL). | LEFT MID DOSING SITE: Dermal inflammation, superficial: mild (H). |
| | LEFT HIGH DOSING SITE: Hyperplasia, epidermal; mild (H) |
| | LEFT HIGH DOSING SITE: Dermal inflammation, superficial: mild (H). |

| RIGHT CONTROL DOSING SITE: | |
|---|---|
| Skin: Dark area; few; red (TGL). | NO CORRELATING LESION: Not correlating with necropsy data (H). |

Any remaining protocol required tissues, which have been examined, have no visible lesions

### Histopathology Observations:

### LEFT LOW DOSING SITE

Hyperplasia, epidermal: minimal: segmental to multifocal, unabreached epidermis
Acanthosis: minimal
Dermal inflammation: superficial; minimal: acute to subacute, multi-focal
Hyperplasia, basal: minimal
Hypergranulosis: minimal

### LEFT MID DOSING SITE

Hyperplasia, epidermal: mild, segmental to multifocal, unabreached epidermis
Dermal inflammation, superficial; mild: acute to subacute, multifocal
Acanthosis: mild;
Hypergranulosis: mild; and
Hyperplasia: basal; mild, with increased mitotic activity.

### LEFT HIGH DOSING SITE

Skin: Dark discoloration (G);
Acanthosis: mild;
Hypergranulosis: mild; and
Hyperplasia: basal; mild, with increased mitotic activity.

### LEFT HIGH DOSING SITE

Hyperplasia: epidermal; mild, and Segmental to multifocal, unabreached epidermis.
Skin: Dark discoloration (G); and
Dermal inflammation: superficial; mild: acute to subacute, multifocal.
Acanthosis: mild;
Hyperplasia: basal; mild: with increased mitotic activity; and
Hypergranulosis: mild

### RIGHT HIGH DOSING SITE

Hyperplasia: epidermal; minimal: segmental, unabreached epidermis
Acanthosis: minimal
Hyperplasia: basal; minimal
Hypergranulosis: minimal

### NO CORRELATING LESION

Not correlating with necropsy data

### RIGHT CONTROL DOSING SITE

Skin; Dark area; few; red (G)

The following tissues were within normal limits: left control dosing site; right control dosing site; right low dosing site; and right mid dosing site.

Codes Used: (G) = Gross Finding; (TGL) = Trackable Gross Lesion; and (H) = Histo Finding

### INDIVIDUAL ANIMAL DATA (Animal Ref.: 1002A)

Group: 1
Sex: Male
Species: Rabbit
Strain: New Zealand White
Test Material: Dose: Group 1 Route: Dermal Study Type: Tolerance Study Date of Death: 12/01/11 Study Day No. (Week): 8 (2) Mode of Death: Terminal Date of Necropsy: 12/01/11 ** NECROPSY COMPLETE **
** EXAMINATION COMPLETE **
Terminal Body Weight: 3kg

| **Gross Pathology Observations** | **Correlated with** |
|---|---|
| LEFT HIGH DOSING SITE | |
| Skin; Dark discoloration (TGL) | LEFT HIGH DOSING SITE: Hyperplasia, epidermal, moderate (H) |
| | LEFT HIGH DOSING SITE: Dermal inflammation, superficial; mild (H) |
| RIGHT HIGH DOSING SITE: Skin; Dark discoloration (TGL) | NO CORRELATING LESION: Not correlating with necropsy data (H) |

Any remaining protocol required tissues, which have been examined, have no visible lesions.

### Histopathology Observations

### LEFT LOW DOSING SITE;

Hyperplasia, epidermal; minimal: segmental to multifocal, unabreached epidermis Acanthosis; minimal
Dermal inflammation, superficial; mild: acute to subacute, multifocal
Hyperplasia, basal; minimal
Hypergranulosis; minimal

### LEFT MID DOSING SITE;

Hyperplasia, epidermal; mild: segmental to multifocal, unabreached epidermis
Dermal inflammation, superficial; mild: acute to subacute, multifocal
Acanthosis; mild
Hypergranulosis; mild
Hyperplasia, basal; mild

### LEFT HIGH DOSING SITE;

Hyperplasia, epidermal; moderate: segmental to multifocal, unabreached epidermis

### LEFT HIGH DOSING SITE;

Skin; Dark discoloration (G)
Dermal inflammation, superficial; mild: acute to subacute, multifocal

### LEFT HIGH DOSING SITE;

Skin; Dark discoloration (G)
Acanthosis; moderate
Hyperplasia,
basal; moderate:
with increased
mitotic activity
Hypergranulosis;
moderate

### NO CORRELATING LESION;

Not correlating with necropsy data

### RIGHT HIGH DOSING SITE;

Skin; Dark discoloration (G)

The following tissues were within normal limits: left control dosing site; right control dosing site; right low dosing site; right mid dosing site; and right high dosing site.

Codes Used: (G) = Gross Finding; (TGL) = Trackable Gross Lesion; and (H) = Histo Finding.

### INDIVIDUAL ANIMAL DATA (Animal Ref.: 1003A)

Group: 1
Sex: Male
Species: Rabbit
Strain: New Zealand White
Test Material: Dose: Group 1 Route: Dermal Study Type: Tolerance Study Date of Death: 12/01/11 Study Day No. (Week): 8 (2) Mode of Death: Terminal Date of Necropsy: 12/01/11 ** NECROPSY COMPLETE **
** EXAMINATION COMPLETE **
Terminal Body Weight: 3.1 kg

| **Gross Pathology Observations** | **Correlated with:** |
|---|---|
| LEFT HIGH DOSING SITE | |
| Skin; Dark discoloration (TGL) | LEFT HIGH DOSING SITE: Hyperplasia, epidermal; mild (H) |
| | LEFT HIGH DOSING SITE: Dermal inflammation, superficial; minimal (H) |

Any remaining protocol required tissues, which have been examined, have no visible lesions.

### Histopathology Observations:

### LEFT LOW DOSING SITE;

Hyperplasia, epidermal; minimal: segmental to multifocal, unabreached epidermis Acanthosis; minimal
Dermal inflammation, superficial; minimal: acute to subacute, multi-focal Hyperplasia, basal; minimal
Hypergranulosis; minimal

### LEFT MID DOSING SITE;

Hyperplasia, epidermal; minimal: segmental to multifocal, unabreached epidermis
Dermal inflammation, superficial; minimal: acute to subacute, multi-focal Acanthosis; minimal
Hypergranulosis; minimal
Hyperplasia, basal; minimal

### LEFT HIGH DOSING SITE;

Hyperplasia, epidermal; mild: segmental to multifocal, unabreached epidermis

### LEFT HIGH DOSING SITE;

Skin; Dark discoloration (G)
Dermal inflammation, superficial; minimal: acute to subacute, multi- focal

### LEFT HIGH DOSING SITE;

Skin; Dark discoloration (G)
Acanthosis; mild
Hyperplasia, basal; mild
Hypergranulosis; mild

### RIGHT HIGH DOSING SITE;

acute to subacute, multifocal
Hyperplasia, epidermal; mild: segmental to multifocal, unabreached epidermis Acanthosis; mild
Hyperplasia, basal; mild
Hypergranulosis; mild
Dermal inflammation, superficial; minimal

The following tissues were within normal limits: left control dosing site; right control dosing site; right low dosing site; and right mid dosing site.

Codes Used: (G) = Gross Finding; (TGL) = Trackable Gross Lesion; and (H) = Histo Finding.

### INDIVIDUAL ANIMAL DATA (Animal Ref.: 1004A)

Group: 1
Sex: Male
Species: Rabbit
Strain: New Zealand White
Test Material: Dose: Group 1 Route: Dermal Study Type: Tolerance Study
Date of Death: 17/01/11 Study Day No. (Week): 13 (2) Mode of Death: Terminal Date of Necropsy: 17/01/11 ** NECROPSY COMPLETE **
** EXAMINATION COMPLETE **
Terminal Body Weight: 2.9 kg

### Gross Pathology Observations: None

Any remaining protocol required tissues, which have been examined, have no visible lesions.

### Histo Pathology Observations: None

The following tissues were within normal limits: left control dosing site; left low dosing site; left mid dosing site; left high dosing site; right control dosing site; right low dosing site; right mid dosing site; and right high dosing site.

### INDIVIDUAL ANIMAL DATA (Animal Ref.: 1005A)

Group: 1
Sex: Male
Species: Rabbit
Strain: New Zealand White
Test Material: Dose: Group 1 Route: Dermal Study Type: Tolerance Study
Date of Death: 17/01/11 Study Day No. (Week): 13 (2) Mode of Death: Terminal Date of Necropsy: 17/01/11 ** NECROPSY COMPLETE **
** EXAMINATION COMPLETE **
Terminal Body Weight: 3.1kg

### Gross Pathology Observations: None

Any remaining protocol required tissues, which have been examined, have no visible lesions.

### Histo Pathology Observations: None

The following tissues were within normal limits: left control dosing site; left low dosing site; left mid dosing site; left high dosing site; right control dosing site; right low dosing site; right mid dosing site; and right high dosing site.

### INDIVIDUAL ANIMAL DATA (Animal Ref.: 1006A)

Group: 1
Sex: Male
Species: Rabbit
Strain: New Zealand White
Test Material: Dose: Group 1 Route: Dermal Study Type: Tolerance Study
Date of Death: 17/01/11 Study Day No. (Week): 13 (2) Mode of Death: Terminal Date of Necropsy: 17/01/11 ** NECROPSY COMPLETE **
** EXAMINATION COMPLETE **
Terminal Body Weight: 3.1 kg

### Gross Pathology Observations: None

Any remaining protocol required tissues, which have been examined, have no visible lesions.

### Histopathology Observations: None

The following tissues were within normal limits: left control dosing site; left low dosing site; left mid dosing site; left high dosing site; right control dosing site; right low dosing site; right mid dosing site; and right high dosing site.

While preferred embodiments have been shown and described herein, it will be obvious to those skilled in the art that such embodiments are provided by way of example only. Numerous variations, changes, and substitutions will now occur to those skilled in the art without departing from the embodiments. It should be understood that various alternatives to the embodiments described herein may be employed in practicing the methods described herein. It is intended that the following claims define the scope of the embodiments and that methods and structures within the scope of these claims and their equivalents be covered thereby.

## Claims

1. A topical pharmaceutical composition for use in wound healing by inducing a favorable epidermal physiological response, comprising digestive enzymes and optionally one or more excipients, wherein said digestive enzymes comprise a protease, a lipase, and an amylase, wherein the ratio (in U.S.P. units) of protease to lipase to amylase is 7: 1: 4 and wherein said composition comprises a sufficient amount of said protease, said lipase and said amylase to induce a favorable epidermal physiological response.

2. The topical pharmaceutical composition for use of claim 1, further comprising one or more enzymes selected from the group consisting of a cellulase, a sucrase, and a maltase.

3. The topical pharmaceutical composition for use of claim 1 or 2, wherein said topical pharmaceutical composition is not used for treating a *Staphylococcus aureus* (*S. aureus*) or *Escherichia coli* (*E. coli*) infection.

4. The topical pharmaceutical composition for use of any one of claims 1-3, wherein said topical pharmaceutical composition is pancreatin.

5. The topical pharmaceutical composition for use of any one of claims 1-4, wherein said topical pharmaceutical composition comprises one or more protease comprising chymotrypsin and trypsin.

6. The topical pharmaceutical composition for use of any one of claims 1-5, wherein said topical pharmaceutical composition stimulates epidermal cells, causes short term fibrosis deposits, prevents re-opening of wounds, recruits white blood cells to help growth factor and immune system activation, induces greater re-growth of hair, reduces alopecia, enhances epidermal restoration and integrity beyond that of the normal restorative process, or a combination thereof.

7. The topical pharmaceutical composition for use of any one of claims 1-6, wherein said epidermal physiological response comprises epidermal hyperplasia, short term fibrosis deposits, recruitment of white blood cells and/or immune system activation.

8. The topical pharmaceutical composition for use of any one of claims 1-7, wherein said topical pharmaceutical composition does not cause an allergic reaction, scarring, biological damage, burns, or a combination thereof.

9. The topical pharmaceutical composition for use of any one of claims 1-8, wherein said topical pharmaceutical composition is a dosage formulation selected from the group consisting of a cream, a lotion, an emulsion, a powder, a liquid, a gel, and a combination of thereof.

10. The topical pharmaceutical composition for use of any one of claims 1-9, wherein said one or more excipients comprises water, saline, Ringer's solution, dextrose, ethanol, glucose, sucrose, dextran, mannose, mannitol, sorbitol, polyethylene glycol (PEG), phosphate, acetate, gelatin, collagen, CARBOPOL®, vegetable oils, white petrolatum, or a combination thereof.

11. The topical pharmaceutical composition for use of any one of claims 1-10, wherein said topical pharmaceutical composition further comprises one or more suitable preservatives, stabilizers, antioxidants, antimicrobials, buffering agents, or a combination thereof.

12. The topical pharmaceutical composition for use of any one of claims 1-11, wherein said digestive enzymes comprise 122,130 U.S.P. units protease, 17,110 U.S.P. units lipase, and 73,750 U.S.P. units amylase in a base of 30 grams of white petrolatum.

13. The topical pharmaceutical composition for use of any one of claims 1-11, wherein said digestive enzymes comprise 238,050 U.S.P. units protease, 33,350 U.S.P. units lipase, and 143,750 U.S.P. units amylase in a base of 30 grams of white petrolatum.

14. The topical pharmaceutical composition for use of any one of claims 1-11, wherein said digestive enzymes comprise 459,540 U.S.P. units protease, 64,380 U.S.P. units lipase, and 277,500 U.S.P. units amylase in a base of 30 grams of white petrolatum.

## Patentansprüche

1. Topische pharmazeutische Zusammensetzung zur Verwendung in der Wundheilung durch Induzieren einer günstigen epidermalen physiologischen Reaktion, die Verdauungsenzyme und optional einen oder mehrere Arzneiträger umfasst, wobei die Verdauungsenzyme eine Protease, eine Lipase und eine Amylase umfassen, wobei das Verhältnis (in USP-Einheiten) von Protease zu Lipase und Amylase 7: 1: 4 ist, und wobei die Zusammensetzung eine ausreichende Menge der Protease, der Lipase und der Amylase umfasst, um eine günstige epidermale physiologische Reaktion zu induzieren.

2. Topische pharmazeutische Zusammensetzung zur Verwendung nach Anspruch 1, die ferner ein oder mehrere Enzyme umfasst, die ausgewählt sind aus der Gruppe bestehend aus einer Cellulase, einer Sucrase und einer Maltase.

3. Topische pharmazeutische Zusammensetzung zur Verwendung nach Anspruch 1 oder 2, wobei die topische pharmazeutische Zusammensetzung nicht zur Behandlung einer *Staphylococcus aureus* (*S. aureus*) oder einer *Escherichia coli* (*E. coli*) Infektion verwendet wird.

4. Topische pharmazeutische Zusammensetzung zur Verwendung nach einem der Ansprüche 1 bis 3, wobei die topische pharmazeutische Zusammensetzung Pankreatin ist.

5. Topische pharmazeutische Zusammensetzung zur Verwendung nach einem der Ansprüche 1 bis 4, wobei die topische pharmazeutische Zusammensetzung eine oder mehrere Proteasen umfassst, die Chymotrypsin und Trypsin umfassen.

6. Topische pharmazeutische Zusammensetzung zur Verwendung nach einem der Ansprüche 1 bis 5, wobei die topische pharmazeutische Zusammensetzung epidermale Zellen stimuliert, kurzfristige Fibroseablagerungen bewirkt, ein erneutes Öffnen von Wunden verhindert, weiße Blutkörperchen rekrutiert zur Unterstützung des Wachstumsfaktors und der Aktivierung des Immunsystems, stärkeres Nachwachsen von Haaren induziert, Alopezie reduziert, die epidermale Erholung und Unversehrtheit über das Ausmaß des normalen Erholungsprozesses hinaus fördert, oder eine Kombination dieser.

7. Topische pharmazeutische Zusammensetzung zur Verwendung nach einem der Ansprüche 1 bis 6, wobei die epidermale physiologische Reaktion epidermale Hyperplasie, kurzfristige Fibroseablagerungen, Rekrutierung weißer Blutkörperchen und/oder eine Aktivierung des Immunsystems umfasst.

8. Topische pharmazeutische Zusammensetzung zur Verwendung nach einem der Ansprüche 1 bis 7, wobei die topische pharmazeutische Zusammensetzung keine allergische Reaktion, Narbenbildung, biologische Schädigung, Verbrennungen oder eine Kombination davon verursacht.

9. Topische pharmazeutische Zusammensetzung zur Verwendung nach einem der Ansprüche 1 bis 8, wobei die topische pharmazeutische Zusammensetzung eine Darreichungsform ist, die ausgewählt ist aus der Gruppe bestehend aus einer Creme, einer Lotion, einer Emulsion, einem Pulver, einer Flüssigkeit, einem Gel und einer Kombination dieser.

10. Topische pharmazeutische Zusammensetzung zur Verwendung nach einem der Ansprüche 1 bis 9, wobei der eine oder die mehreren Arzneiträger folgendes umfassen: Wasser, Kochsalzlösung, Ringerlösung, Dextrose, Ethanol, Glucose, Saccharose, Dextran, Mannose, Mannitol, Sorbitol, Polyethylenglycol (PEG), Phosphat, Acetat, Gelatine, Collagen, CARBOPOL®, Pflanzenöle, Naturvaseline oder eine Kombination davon.

11. Topische pharmazeutische Zusammensetzung zur Verwendung nach einem der Ansprüche 1 bis 10, wobei die topische pharmazeutische Zusammensetzung ferner eines oder mehrere der folgenden umfasst: geeignete Konservierungsstoffe, Stabilisatoren, Antioxidationsmittel, antimikrobielle Substanzen, Puffermittel oder eine Kombination davon.

12. Topische pharmazeutische Zusammensetzung zur Verwendung nach einem der Ansprüche 1 bis 11, wobei die Verdauungsenzyme 122.130 USP-Einheiten Protease, 17.110 USP-Einheiten Lipase und 73.750 USP-Einheiten Amylase in einer Base von 30 Gramm Naturvaseline umfassen.

13. Topische pharmazeutische Zusammensetzung zur Verwendung nach einem der Ansprüche 1 bis 11, wobei die Verdauungsenzyme 238.050 USP-Einheiten Protease, 33.350 USP-Einheiten Lipase und 143.750 USP-Einheiten Amylase in einer Base von 30 Gramm Naturvaseline umfassen.

14. Topische pharmazeutische Zusammensetzung zur Verwendung nach einem der Ansprüche 1 bis 11, wobei die Verdauungsenzyme 459.540 USP-Einheiten Protease, 64.380 USP-Einheiten Lipase und 277.500 USP-Einheiten Amylase in einer Base von 30 Gramm Naturvaseline umfassen.

## Revendications

1. Composition pharmaceutique topique destinée à être utilisée dans la cicatrisation des plaies en induisant une réponse physiologique épidermique favorable, comprenant des enzymes digestives et éventuellement au moins un excipient, lesdites enzymes digestives comprenant une protéase, une lipase et une amylase, le rapport (en unités U.S.P.) de la protéase à la lipase à l'amylase étant de 7:1:4 et ladite composition comprenant une quantité suffisante de ladite protéase, de ladite lipase et de ladite amylase pour induire une réponse physiologique épidermique favorable.

2. Composition pharmaceutique topique destinée à être utilisée selon la revendication 1, comprenant en outre au moins une enzyme choisie dans le groupe constitué par une cellulase, une sucrase et une maltase.

3. Composition pharmaceutique topique destinée à être utilisée selon la revendication 1 ou 2, ladite composition pharmaceutique topique n'étant pas utilisée pour traiter une infection par *Staphylococcus aureus* (*S. aureus*) ou *Escherichia coli* (*E. coli*)*.*

4. Composition pharmaceutique topique destinée à être utilisée selon l'une quelconque des revendications 1 à 3, ladite composition pharmaceutique topique étant la pancréatine.

5. Composition pharmaceutique topique destinée à être utilisée selon l'une quelconque des revendications 1 à 4, ladite composition pharmaceutique topique comprenant au moins une protéase comprenant la chymotrypsine et la trypsine.

6. Composition pharmaceutique topique destinée à être utilisée selon l'une quelconque des revendications 1 à 5, ladite composition pharmaceutique topique stimulant les cellules épidermiques, provoquant des dépôts de fibrose à court terme, empêchant la réouverture des plaies, recrutant des globules blancs pour aider à l'activation du facteur de croissance et du système immunitaire, induisant une plus grande repousse des cheveux, réduisant l'alopécie, améliorant la restauration et l'intégrité de l'épiderme au-delà du processus normal de restauration, ou une combinaison de ces éléments.

7. Composition pharmaceutique topique destinée à être utilisée selon l'une quelconque des revendications 1 à 6, ladite réponse physiologique épidermique comprenant une hyperplasie épidermique, des dépôts de fibrose à court terme, le recrutement de globules blancs et/ou l'activation du système immunitaire.

8. Composition pharmaceutique topique destinée à être utilisée selon l'une quelconque des revendications 1 à 7, ladite composition pharmaceutique topique ne provoquant pas de réaction allergique, de cicatrices, de dommages biologiques, de brûlures, ou une combinaison de celles-ci.

9. Composition pharmaceutique destinée à être utilisée selon l'une quelconque des revendications 1 à 8, la composition pharmaceutique topique étant une forme galénique choisie dans le groupe constitué par une crème, une lotion, une émulsion, une poudre, un liquide, un gel et toute combinaison associée.

10. Composition pharmaceutique topique destinée à être utilisée selon l'une quelconque des revendications 1 à 9, ledit au moins un excipient comprenant de l'eau, une solution saline, une solution de Ringer, du dextrose, de l'éthanol, du glucose, du saccharose, du dextrane, du mannose, du mannitol, du sorbitol, du polyéthylène glycol (PEG), du phosphate, de l'acétate, de la gélatine, du collagène, du CARBOPOL®, des huiles végétales, du pétrolatum blanc ou une combinaison de ceux-ci.

11. Composition pharmaceutique topique destinée à être utilisée selon l'une quelconque des revendications 1 à 10, ladite composition pharmaceutique topique comprenant en outre des conservateurs, des stabilisateurs, des antioxydants, des antimicrobiens, des tampons appropriés et/ou une combinaison de ceux-ci.

12. Composition pharmaceutique topique destinée à être utilisée selon l'une quelconque des revendications 1 à 11, lesdites enzymes digestives comprenant 122 130 unités U.S.P. de protéase, 17 110 unités U.S.P. de lipase et 73 750 unités U.S.P. d'amylase dans une base de 30 grammes de pétrolatum blanc.

13. Composition pharmaceutique topique destinée à être utilisée selon l'une quelconque des revendications 1 à 11, lesdites enzymes digestives comprenant 238 050 unités U.S.P. de protéase, 33 350 unités U.S.P. de lipase et 143 750 unités U.S.P. d'amylase dans une base de 30 grammes de pétrolatum blanc.

14. Composition pharmaceutique topique destinée à être utilisée selon l'une quelconque des revendications 1 à 11, lesdites enzymes digestives comprenant 459 540 unités U.S.P. de protéase, 64 380 unités U.S.P. de lipase et 277 500 unités U.S.P. d'amylase dans une base de 30 grammes de pétrolatum blanc.
